# EUROPEAN PATENT APPLICATION

(11) **EP 4 818 172 A1**
(43) Date of publication of application: **07.10.2026**
(21) Application number: 24896964.4
(22) Date of filing: 05.07.2024
(51) Int. Cl.: C07D 493/04, A61K 8/49, A61K 8/67, A61K 31/357, A61P 17/00, A61P 19/00, A61P 43/00, A61Q 19/00, A61Q 19/02, C07D 493/14

(54) **ASCORBIC ACID DERIVATIVES AND COSMETIC CONTAINING SAME**

(30) Priority: 28.11.2023 JP 2023201078; 14.02.2024 JP 2024019991
(71) Applicant: Seiwa Kasei Co., Ltd., Higashiosaka-shi, Osaka 579-8004 (JP)
(72) Inventor: YOSHIOKA, Masato, Higashiosaka-shi, Osaka 579-8004 (JP); TSUBOI, Tatsuya, Higashiosaka-shi, Osaka 579-8004 (JP); HONDA, Tasuku, Higashiosaka-shi, Osaka 579-8004 (JP); IWAKI, Nobuyasu, Higashiosaka-shi, Osaka 579-8004 (JP); TOMIYAMA, Ai, Higashiosaka-shi, Osaka 579-8004 (JP)
(74) Representative: Vossius & Partner Patentanwälte Rechtsanwälte mbB
(86) International application number: PCT/JP2024/024351
(87) International publication number: WO 2025/115272

(57) **Abstract**

Provided are novel ascorbic acid derivatives which have the excellent functions inherent to ascorbic acid, such as collagen production promoting effect and moisturizing effect, and which are stable even when stored for a long period of time and show little decrease in activity, as well as cosmetics, collagen production promoters and hyaluronic acid production promoters containing the ascorbic acid derivative, wherein the ascorbic acid derivatives are represented by the following general formula (1), (3) or (4):

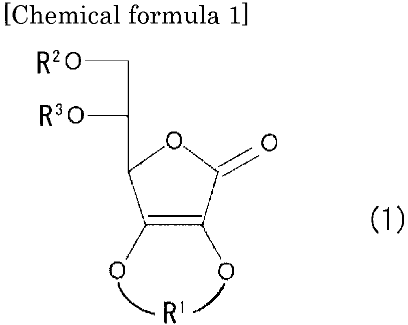

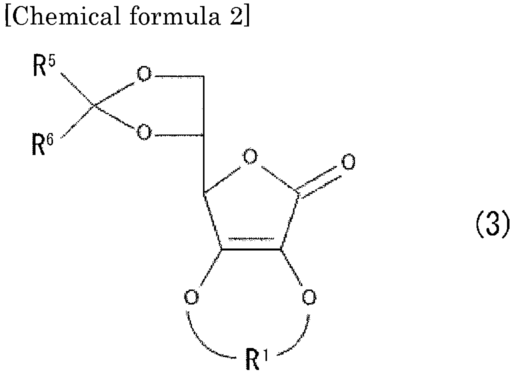

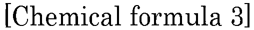

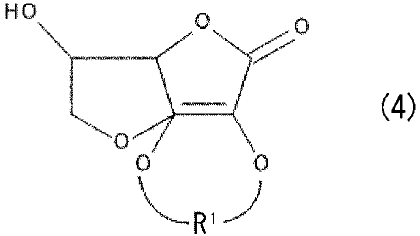

[In formula (1), (3), or (4), R¹ is -(CH₂) ₙ -* (n is an integer of 2 to 4),-CH₂ CH(OH)CH₂ -* (* represents the position of bonding to the O at the 2nd position of the ascorbic ring), or a bismethyloxetane group; R² and R³ are hydrogen, -COR⁴, an alkyl group, or a benzyl group; R⁴ is an alkyl group having 1 to 22 carbon atoms; and in formula (3), R⁵ and R⁶ are hydrogen, a methyl group, or a phenyl group.].

## Description

### TECHNICAL FIELD

The present invention relates to an ascorbic acid derivative that is suitable for use as a raw material for cosmetics, and further to a cosmetic containing the ascorbic acid derivative.

### BACKGROUND ART

Ascorbic acid is a safe and useful antioxidant and is known as a compound with excellent whitening properties. However, its use in the cosmetics field has been hindered due to its instability to light, heat, and oxidation, and its insufficient stability over time. Therefore, various ascorbic acid derivatives or salts thereof have been proposed as derivatives with improved stability over time compared to ascorbic acid, and their incorporation into topical skin whitening preparations (Patent Documents 1 and 2) and into cosmetics (Patent Document 3) have been proposed.

However, many of the above-mentioned ascorbic acid derivatives and their salts still have insufficient stability over time, and have problems such as a decrease in pH in the formulation over time, accelerated decomposition under alkaline conditions, etc. Furthermore, the duration of activity in vivo is not sufficient, and improvements in this respect are desired.

The present inventors have solved these problems by proposing an ascorbic acid derivative that is inhibited from decomposing under alkaline conditions (Patent Document 4). However, there is a need for an ascorbic acid derivative that has further improved stability over time under alkaline conditions and high stability over a wide pH range.

### PRIOR ART DOCUMENT

### Patent Document

| | |
|---|---|
| Patent Document 1: | JP 1987-221611 A |
| Patent Document 2: | JP 2005-060239 A |
| Patent Document 3: | JP 1989-228978 A |
| Patent Document 4: | JP 4681670 B1 |

### SUMMARY OF THE INVENTION

### PROBLEM TO BE SOLVED BY THE INVENTION

An object of the present invention is to provide an ascorbic acid derivative that not only has the excellent properties inherent to ascorbic acid, such as moisturizing property, but also has high stability over a wide pH range and excellent physiologically active effects. A further object of the present invention is to provide cosmetics and pharmaceuticals that contain the ascorbic acid derivative and exhibit excellent moisturizing activity, etc., as well as high stability over a wide pH range and excellent physiologically active effects.

### MEANS FOR SOLVING THE PROBLEM

The present inventors have conducted extensive studies in light of the above-mentioned circumstances, and have found that novel ascorbic acid derivatives represented by the following formula (1), (3) or (4) have excellent moisturizing properties, and some of them are highly stable over a wide pH range, and have excellent physiologically active effects such as the effect of promoting hyaluronic acid production. Furthermore, they have found that cosmetics and pharmaceuticals containing the ascorbic acid derivatives represented by the following formula (1), (3) or (4) have excellent moisturizing properties, are stable over a wide pH range, and further have excellent physiologically active effects such as a hyaluronic acid production promoting effect. The present invention has been completed based on these findings.

The first aspect of the present invention is an ascorbic acid derivative represented by the following general formula (1), (3) or (4) (claim 1).

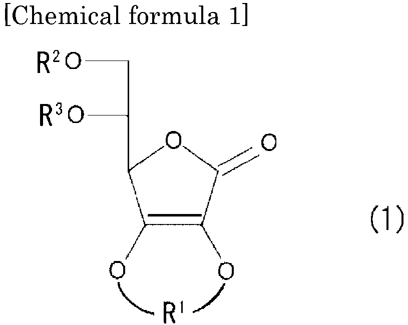

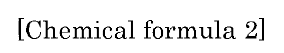

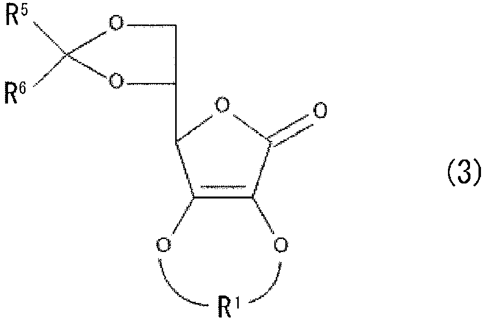

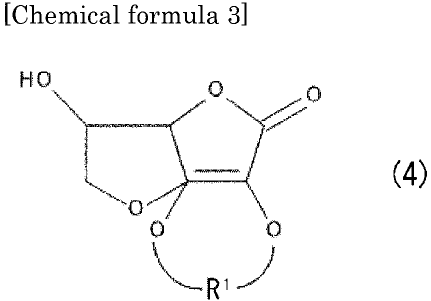

[In formula (1), (3) or (4), R¹ is -(CH₂)ₙ -* (n is an integer of 2 to 4), - CH₂ CH(OH)CH₂ -*, -CH₂ CH(CH₂ OH)-*, -CH(CH₂ OH)CH₂ -*, -C(CH₃ )₂ CH₂ - *, -CH₂ C(CH₃ )₂ -* (* represents the position bonding to O at the 2-position of the ascorbic ring), or a divalent group represented by the following formula (2);
in formula (1), R² and R³ are each hydrogen, -COR⁴, a linear or branched alkyl group having 1 to 22 carbon atoms, or a benzyl group, and R⁴ is a linear or branched alkyl group having 1 to 22 carbon atoms; and
in formula (3), R⁵ and R⁶ are each a hydrogen atom, a methyl group, or a phenyl group.]

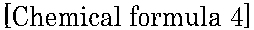

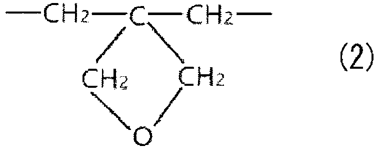

In the formulas (1), (3), and (4), carbon atoms and hydrogen atoms bonded to the carbon atoms are omitted. For example, in formula (1), positions 1, 2, and 3 are carbon atoms, positions 4 and 5 are CH groups, and position 6 is a CH₂ group. In the following structural formulas, hydrogen atoms and carbon atoms are omitted, as in these formulas.

The ascorbic acid derivatives represented by the general formula (1) have superior stability over time in the neutral to weakly alkaline range (pH range of about 6 to 10) compared to ascorbic acid and conventional ascorbic acid derivatives. Particularly, the ascorbic acid derivatives maintain a high residual rate over a wide pH range even when stored for several weeks in a high-temperature environment above room temperature, and suppress problems such as odor generation and discoloration. Furthermore, the ascorbic acid derivatives represented by general formula (1), (3) or (4) have superior collagen production promoting effects and hyaluronic acid production promoting effects compared to conventional ascorbic acid derivatives such as glyceryl ascorbic acid.

The second aspect of the present invention is a preferred embodiment of the first aspect of the present invention, which is an ascorbic acid derivative represented by the general formula (1), wherein R¹ in formula (1) is -(CH₂) n-* (n is 3 or 4), -CH₂CH( OH)CH₂-* , -CH₂CH(CH₂OH) - *, - CH(C H ₂ O H )CH₂-* (* represents the position bonding to the O at the 2-position of the ascorbic acid ring), or a divalent group represented by formula (2), and R² and R³ are hydrogen; or R¹ is -(CH₂) n-* (n is 3 or 4), R² is -COR⁴, and R³ is hydrogen or -COR⁴, and R⁴ is a linear or branched alkyl group having 4 to 18 carbon atoms. These compounds are preferred because they have particularly excellent stability over time in the neutral to weakly alkaline range.

The third aspect of the present invention is a preferred embodiment of the second aspect of the present invention, which is an ascorbic acid derivative represented by the general formula (1),
wherein R¹ in general formula (1) is -(CH₂) ³-* , -CH₂CH(OH)CH₂-* (* represents the position bonding to the O at position 2 of the ascorbic acid ring), or a divalent group represented by the formula (2), and R² and R³ are each hydrogen, or R¹ is -(CH₂)₃-*, R² is -COR⁴, R³ is hydrogen or -COR⁴, and R⁴ is a linear or branched alkyl group having 8 to 18 carbon atoms. These compounds are preferred in that they have particularly excellent effects of promoting collagen production and hyaluronic acid production. Among these, ascorbic acid derivatives characterized in that R¹ is -(CH₂)₃-* or a divalent group represented by the formula (2), and R² and R³ are hydrogen, or R¹ is -(CH₂)₃-*, R² is -COR⁴, R³ is hydrogen or -COR⁴, and R⁴ is a linear or branched alkyl group having 8 to 18 carbon atoms are particularly preferred, because they have a particularly excellent effect of promoting hyaluronic acid production.

The ascorbic acid derivative of the present invention can be blended into cosmetics. A fourth aspect of the present invention is a cosmetic wherein the ascorbic acid derivative of any one of the first to third aspects of the present invention is blended. The fourth aspect of the present invention is a cosmetic that has excellent moisturizing properties, is stable over a wide pH range, and exhibits excellent physiologically active effects such as a collagen production promoting effect and a hyaluronic acid production promoting effect.

The fifth aspect of the present invention is a collagen production promoter comprising the ascorbic acid derivative of the third aspect of the present invention. The collagen production promoter of the fifth aspect of the present invention is stable over a wide pH range, exhibits excellent physiological activity, and exhibits a particularly excellent collagen production promoting effect, so it is suitable for use as a collagen production promoter.

The sixth aspect of the present invention is a hyaluronic acid production promoter, comprising the ascorbic acid derivatives of the first to third aspects of the present invention. The sixth aspect of the present invention is stable in a wide pH range, has excellent physiological activity effects, and exhibits excellent hyaluronic acid production promoting effects, so it is suitable for use as a hyaluronic acid production promoter.

Among the hyaluronic acid production promoters of the sixth aspect of the present invention, those characterized by comprising the ascorbic acid derivative of the third aspect of the present invention have particularly excellent hyaluronic acid production promoting effects, and are therefore even more preferably used as hyaluronic acid production promoters.

### EFFECT OF THE INVENTION

The ascorbic acid derivatives of the present invention represented by the general formula (1), (3) or (4) have the excellent functions that ascorbic acid originally has, such as moisturizing effect, are stable in a wide pH range even after long-term storage, with little discoloration, odor change, or activity reduction, and have high physiological activities such as collagen production promoting effect and hyaluronic acid production promoting effect. Therefore, by blending the ascorbic acid derivatives represented by the general formula (1), (3) or (4) into cosmetics such as skin external preparations and hair cosmetics, cosmetics with excellent moisturizing effect, stable even after long-term storage, and high physiological activities, such as moisturizing cosmetics, can be provided. Furthermore, by blending the ascorbic acid derivatives represented by the general formula (1), (3) or (4), a collagen production promoter that exhibits excellent collagen production promoting effect and a hyaluronic acid production promoter that exhibits excellent hyaluronic acid production promoting effect can be provided.

### MODES FOR CARRYING OUT THE INVENTION

The following describes an embodiment of the present invention, but the scope of the present invention is not limited to the embodiment described below.

Specific examples of the ascorbic acid derivatives represented by general formula (1), (3) or (4) include 2,3-0-(1,2-ethanediyl)ascorbic acid, 2,3-O-(1,3-propanediyl)ascorbic acid, 2,3-0-(1,4-butanediyl)ascorbic acid, 2,3-O-(2-hydroxypropane-1,3-diyl)ascorbic acid, 2,3-O-(1-hydroxymethyl-1,2-ethanediyl)ascorbic acid, 2,3-O-(2-hydroxymethyl-1,2-ethanediyl)ascorbic acid, 2,3-O-(3,3-dimethyleneoxetane)ascorbic acid, 2,3-O-(2,2-dimethyl-1,2-ethanediyl)ascorbic acid, 2,3-0-(1,1-dimethyl-1,2-ethanediyl)ascorbic acid,
6-O-butanoyl-2,3-O-(1,4-butanediyl) ascorbic acid, 5,6-O-dibutanoyl-2,3-O-(1,4-butanediyl) ascorbic acid, 6-O-octanoyl-2,3-O-(1,4-butanediyl) ascorbic acid, 5,6-O-dioctanoyl-2,3-O-(1,4-butanediyl) ascorbic acid, 6-O-lauroyl-2,3-O-(1,4-butanediyl) ascorbic acid, 5,6-O-dilauroyl-2,3-O-(1,4-butanediyl) ascorbic acid, 6-O-palmitoyl-2,3-O-(1,4-butanediyl)ascorbic acid, 5,6-O-dipalmitoyl-2,3-O-(1,4-butanediyl)ascorbic acid, 6-O-isostearoyl-2,3-O-(1,4-butanediyl)ascorbic acid, 5,6-O-diisostearoyl-2,3-O-(1,4-butanediyl)ascorbic acid, 5-O-butanoyl-6-O-octanoyl-2,3-O-(1,4-butanediyl)ascorbic acid,
6-O-butanoyl-2,3-O-(1,3- propanediyl) ascorbic acid, 5,6-O-dibutanoyl-2,3-O-(1,3- propanediyl) ascorbic acid, 6-O-octanoyl-2,3-O-(1,3-propanediyl) ascorbic acid, 5,6-O-dioctanoyl-2,3-O-(1,3- propanediyl) ascorbic acid, 6-O-lauroyl-2,3-O-(1,3- propanediyl) ascorbic acid, 5,6-O-dilauroyl-2,3-0-(1,3- propanediyl) ascorbic acid, 6-O-palmitoyl-2,3-O-(1,3- propanediyl) ascorbic acid, 5,6-O-dipalmitoyl-2,3-O-(1, 3-propanediyl) ascorbic acid, 6-O-isostearoyl-2,3-O-(1,3- propanediyl) ascorbic acid, 5,6-O-diisostearoyl-2,3-O-(1,3- propanediyl) ascorbic acid, 5-butanoyl-6-O-palmitoyl-2,3-O-(1,3-propanediyl) ascorbic acid,
6-O-isostearoyl-2,3-O-(1,2- ethanediyl) ascorbic acid, 5,6-O-diisostearoyl-2,3-O-(1,2- ethanediyl) ascorbic acid,
5,6-O-isopropylidene-2,3-O-(1,3-propanediyl) ascorbic acid, 5,6-O-isopropylidene-2,3-O-(1,4-butanediyl) ascorbic acid,
5,6-O-(phenylmethylene)-2,3-O-(1,3-propanediyl) ascorbic acid, 5,6-O-(phenylmethylene)-2,3-O-(1,4-butanediyl) ascorbic acid, 4-hydroxy-2,6,9,14-tetraoxatricyclo[6.6.0.0^{1.5}]tetradecan-7-one, 4-hydroxy-2,6,9,13-tetraoxatricyclo[6.5.0.0^{1.5} ]tridecan-7-one, and 6-O-benzyl-2,3-O-(1,4-butanediyl) ascorbic acid.

The ascorbic acid derivatives represented by the general formula (1), (3) or (4) can be produced by various methods.

For example, a dihaloalkane, 2-halomethyloxirane, bishalomethyloxetane, or the like can be reacted with the hydroxyl groups at the 2- and 3-positions of ascorbic acid to form a cyclic structure from the hydroxyl groups, and then, by known means, acylation, alkylation, benzylation, or acetalization of the oxygen atoms bonded to the 5- and 6-positions can be performed to obtain an ascorbic acid derivative represented by the general formula (1) or (3). Alternatively, the oxygen atoms bonded to the 5- and 6-positions of ascorbic acid can be acylated, alkylated, benzylated, or acetalized by known means, and then, by known means, dihaloalkane, or the like can be used to form a cyclic structure from the hydroxyl groups at the 2- and 3-positions to obtain an ascorbic acid derivative represented by the general formula (1) or (3).

The ascorbic acid derivative represented by general formula (4) can be obtained by forming a cyclic structure from the hydroxyl groups at the 2- and 3-positions of ascorbic acid as described above, followed by an intramolecular reaction in the presence of a strong alkali and then a strong acid.

Examples of compounds capable of reacting with the hydroxyl groups at the 2- and 3-positions of ascorbic acid to form a cyclic structure include, but are not limited to, dihaloalkanes, 2-halomethyloxiranes, and bishalomethyloxetanes.

Examples of dihaloalkanes include dibromoethane, dibromopropane, dibromobutane, dichloroethane, dichloropropane, dichlorobutane, diiodoethane, diiodopropane, and diiodobutane.

Examples of 2-halomethyloxirane include 2-bromomethyloxirane, 2-chloromethyloxirane, and 2-iodomethyloxirane.

Examples of the bishalomethyloxetane include bisbromomethyloxetane and bischloromethyloxetane.

In the synthesis of the ascorbic acid derivative represented by general formula (1), (3), or (4), the amount of dihaloalkane, 2-halomethyloxirane, or bishalomethyloxetane used to form a cyclic structure by reacting with the hydroxyl groups at the 2- and 3-positions of ascorbic acid is not particularly limited, but is preferably 0.5 to 2.0 mol, more preferably 1.0 to 1.5 mol, per 1 mol of ascorbic acid.

Specifically, ascorbic acid derivatives represented by general formula (1), (3), or (4), in which R¹ is -(CH₂) ₙ -* (n is an integer of 2 to 4), can be obtained by reacting ascorbic acid with a dihaloalkane (having 2 to 4 carbon atoms) to form a cyclic structure between the hydroxyl groups at the 2- and 3-positions of ascorbic acid and the dihaloalkane.

Furthermore, ascorbic acid derivatives represented by general formula (1), (3) or (4), in which R¹ is -CH₂ CH(OH)CH₂ -*, or in which R¹ is - CH₂ CH(CH₂ OH) -* or -CH(CH₂ OH)CH₂ -*, can be obtained by reacting ascorbic acid with 2-halomethyloxirane to form a cyclic structure from the hydroxyl groups at the 2- and 3-positions of ascorbic acid.

Furthermore, the ascorbic acid derivatives represented by the general formula (1), (3), or (4), in which R¹ is a divalent group represented by the general formula (2), can be obtained by reacting ascorbic acid with bishalomethyloxetane to form a cyclic structure from the hydroxyl groups at the 2- and 3-positions of ascorbic acid.

The above-described reaction for synthesizing the ascorbic acid derivative represented by general formula (1), (3), or (4) can be carried out in various solvents. Examples of the solvent include water, lower alcohols such as methanol, ethanol, and isopropanol, dimethyl sulfoxide (DMSO), N,N-dimethylformamide (DMF), dioxane, tetrahydrofuran (THF), N-methylpyrrolidone, acetonitrile, and mixtures thereof, and are not particularly limited. The reaction temperature is not particularly limited, but is preferably in the range of 30 to 100°C, more preferably in the range of 50 to 90°C, and particularly preferably in the range of 60 to 90°C.

Examples of pH adjusters used during the reaction include lactic acid, citric acid, glycolic acid, succinic acid, tartaric acid, malic acid, gluconic acid, sodium hydroxide, potassium hydroxide, potassium carbonate, sodium carbonate, sodium hydrogencarbonate, ammonium hydrogencarbonate, triethylamine, diazabicycloundecene, and p-toluenesulfonic acid monohydrate.

From the reaction product of the ascorbic acid derivative represented by general formula (1), (3) or (4) produced as described above, the desired compound can be separated and purified by means of HPLC (liquid chromatography), column chromatography using silica gel, column chromatography using a resin such as an ion exchange resin, activated carbon treatment, extraction, distillation, crystallization, or the like.

Also, ascorbic acid derivatives represented by the general formula (1), in which R¹ is -(CH₂) ₙ -* (n is an integer of 2 to 4), and R² and/or R³ is - COR⁴, a linear or branched alkyl group having 1 to 22 carbon atoms, or a benzyl group;
ascorbic acid derivatives represented by general formula (3), in which R¹ is -(CH₂) ₙ -* (n is an integer of 2 to 4), and R⁵ and/or R⁶ is hydrogen, a methyl group, or a phenyl group; and
ascorbic acid derivatives represented by the general formula (4), in which R¹ is -(CH₂) ₙ -* (n is an integer of 2 to 4);
can be produced by synthesizing an ascorbic acid derivative having a cyclic structure formed from the hydroxyl groups at the 2- and 3-positions, as described above, and then carrying out various known methods.

For example, an ascorbic acid derivative represented by general formula (1) in which R¹ is -(CH₂) ₙ -* (n is an integer of 2 to 4) and R² and /or R³ is -COR⁴ can be synthesized by mixing the ascorbic acid derivative having the cyclic structure with various acid halides or various acid anhydrides, or by mixing the ascorbic acid derivative having the cyclic structure with various carboxylic acids in concentrated sulfuric acid.

Furthermore, ascorbic acid derivatives represented by general formula (1) in which R¹ is -(CH₂) ₙ -* (n is an integer of 2 to 4) and R² is a benzyl group can be produced by synthesizing an ascorbic acid derivative represented by general formula (3) in which R⁵ is a phenyl group and R⁶ is hydrogen, followed by ring-opening using a reducing agent.

Furthermore, an ascorbic acid derivative represented by general formula (3), in which R¹ is -(CH₂) ₙ -* (n is an integer of 2 to 4) and R⁵ and /or R⁶ is hydrogen, a methyl group, or a phenyl group, can be produced by synthesizing an ascorbic acid derivative in which a cyclic structure is formed from the hydroxyl groups at the 2- and 3-positions, and then reacting the resulting compound with a dialkoxyalkyl, α,α-dialkoxytoluene, or the like under strong acid conditions.

Ascorbic acid derivatives represented by general formula (4), in which R¹ is -(CH₂) ₙ -* (n is an integer of 2 to 4), can be obtained by forming a cyclic structure from the 2- and 3-positions of ascorbic acid as described above, followed by an intramolecular reaction with a strong alkali and then a strong acid.

Examples of the acid halide which can be used in the above reaction include acetyl chloride, acetyl bromide, propionyl chloride, propionyl bromide, butanoyl chloride, octanoyl chloride, nonanoyl chloride, decanoyl chloride, undecanoyl chloride, dodecanoyl chloride, tridecanoyl chloride, tetradecanoyl chloride, pentadecanoyl chloride, hexadecanoyl chloride, heptadecanoyl chloride, octadecanoyl chloride, nonadecanoyl chloride, eicosanoyl chloride, henicosanoyl chloride, docosanoyl chloride, 2-ethylhexanoyl chloride, 3-ethylhexanoyl chloride, 5-methylheptanoyl chloride, isostearoyl chloride (for example, 2-octyldecanoyl chloride or 16-methylheptadecanoyl chloride), isononanoyl chloride, isodecanoyl chloride, isoundecanoyl chloride, isododecanoyl chloride, isotridecanoyl chloride, isotetradecanoyl chloride, isopentadecanoyl chloride, isohexadecanoyl chloride, isoheptadecanoyl chloride, isononadecanoyl chloride, isoeicosanoyl chloride, isoheneicosanoyl chloride, and isodocosanoyl chloride.

When an acid halide derived from a branched fatty acid is used among the above acid halides, the commercially available product may contain a mixture of acid halides with different branching points, and thus a reaction product to which a fatty acid with a different branching point is added may be obtained as a by-product. For example, when commercially available 16-methylheptadecanoyl chloride is used, a compound formed by reaction with 2-octyldecanoyl chloride, which has a different branching point, may also be produced as a by-product. In this case, the ascorbic acid derivative of the present invention also contains this by-product.

There is no particular limitation on the amount of the acid halide to be used. However, when the acid halide is mainly introduced into either the 5- or 6- hydroxyl group, the amount is preferably 0.5 to 2.0 mol, and particularly preferably 0.8 to 1.5 mol, per mol of the ascorbic acid derivative having a cyclic structure.

When the amount of acid halide used is 1.5 to 5.0 mol, preferably 1.5 to 3.0 mol, per mol of the ascorbic acid derivative forming a cyclic structure, a mixture of compounds introduced into only one and compounds introduced into both is obtained. Alternatively, after obtaining a compound having either the hydroxyl group at the 5- or 6-position introduced under the above conditions, further reaction with an acid halide or the like can give a compound introduced into both at the 5- and 6-positions.

As the dialkoxyalkyl or α,α-dialkoxytoluene used in the above reaction, dimethoxymethane, dimethoxypropane, α,α-dimethoxytoluene, or the like can be used. There is no particular limitation on the amount of the dialkoxyalkyl or α,α-dialkoxytoluene used, but it is preferably 0.8 to 2.5 mol, and particularly preferably 1.0 to 2.0 mol, per mol of the ascorbic acid derivative having a cyclic structure.

The reaction with an acid halide, a dialkoxyalkyl, or an α,α-dialkoxytoluene can be carried out by adjusting the solvent, reaction temperature, and pH in the same manner as in the reaction for forming a cyclic structure. And the ascorbic acid derivative of the present invention having the desired structure can be obtained by purifying the reaction by means of chromatography using silica gel, column chromatography using a resin such as an ion exchange resin, activated carbon treatment, extraction, distillation, crystallization, or the like.

The ascorbic acid derivative of the present invention can be suitably used as a component of various cosmetics such as external skin preparations and hair cosmetics, and can also be used as a food additive, animal feed, etc.

When the ascorbic acid derivative of the present invention is blended into various cosmetic preparations, the blending amount is preferably 1 to 20% by mass, and particularly preferably 3 to 10% by mass, of the total amount of the cosmetics. If the blending amount is less than 1% by mass, the effects of the ascorbic acid derivative of the present invention, such as the hyaluronic acid production promoting effect, often cannot be fully exerted. On the other hand, if the blending amount is more than 20% by mass, the formulation system may be damaged, and even if the blending amount is increased, improvement in the effect may not be expected in many cases.

In addition to these essential ingredients, the cosmetics of the present invention may contain, as appropriate, commonly used ingredients such as oily raw materials, surfactants, moisturizers, polymeric compounds, antioxidants, whitening agents, pharmaceuticals, ultraviolet absorbers, sequestering agents, proteins, protein hydrolysates or derivatives thereof, amino acids or derivatives thereof, pH adjusters, and preservatives. Although the ascorbic acid derivative of the present invention also exhibits an effect as a moisturizer, other moisturizers may also be blended into the cosmetics of the present invention as appropriate.

Examples of the oily raw materials, surfactants, other moisturizers, polymeric compounds, antioxidants, whitening agents, other drugs, ultraviolet absorbers, sequestering agents, proteins, protein hydrolysates or derivatives thereof, amino acids or derivatives thereof, pH adjusters, and preservatives include those similar to those described in WO2022/080287.

The cosmetics of the present invention may be formulated in any formulation system, including a solution system, a solubilized system, an emulsion system, a gel system, a powder dispersion system, and a water-oil two-layer system. The cosmetics of the present invention can be produced by blending the ascorbic acid derivative represented by the above general formula (1), (3), or (4) with the above optional ingredients according to the desired product.

### Example

Next, specific embodiments for carrying out the present invention will be described in detail with reference to Examples, but the scope of the present invention is not limited to these Examples. Prior to the Examples, examples of the production of the ascorbic acid derivatives of the present invention used in the Examples will be shown as Synthesis Examples.

### Synthesis Example 1

### (Synthesis of 2,3-O-(1,2-ethanediyl) ascorbic acid)

DMF (9.0 g), ascorbic acid (0.88 g), potassium carbonate (0.76 g), and dibromoethane (1.03 g) were added in an eggplant-shaped flask and stirred at 80°C for 3 hours. After cooling, the mixture was filtered and concentrated under reduced pressure. The resulting residue (1.2 g) was subjected to silica gel chromatography and eluted with a mixture of chloroform/methanol/water (20/3/0.3). Further concentration under reduced pressure yielded 2,3-O-(1,2-ethanediyl) ascorbic acid (0.505 g).

The resulting product was subjected to mass spectrometry, ¹ H-NMR and ^{1 3} C-NMR measurements, and the results confirmed that the product was 2,3-O-(1,2-ethanediyl) ascorbic acid represented by the following structural formula.

In the synthesis examples shown below, the products obtained were also subjected to mass spectrometry, ¹ H-NMR, and ^{1 3} C-NMR measurements, and the results of these measurements confirmed that the products were ascorbic acid derivatives represented by the structural formula or compound name shown in each synthesis example (including cases where the products consist of two or more ascorbic acid derivatives), or that the products were mainly composed of the ascorbic acid derivatives. The results of mass spectrometry, ¹ H-NMR, and ^{1 3} C-NMR measurements for the products obtained in each synthesis example are shown in Tables 1 to 9.

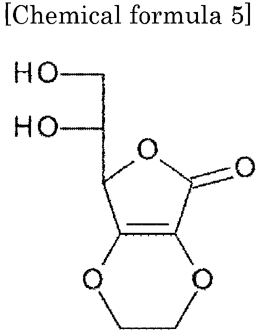

### Synthesis Example 2

### (Synthesis of 2,3-O-(1,3-propanediyl) ascorbic acid)

DMF (35.0 g), ascorbic acid (3.50 g), potassium carbonate (3.0 g), and dibromopropane (4.4 g) were added in an eggplant-shaped flask and stirred at 80°C for 16 hours. After cooling, the mixture was filtered, and the filtrate was concentrated under reduced pressure. The resulting concentrate was dissolved in water. Extraction was performed using isobutyl alcohol, and the organic layer was recovered, to which magnesium sulfate was added. Subsequently, the mixture was filtered, and the filtrate was concentrated under reduced pressure. The resulting residue (4.2 g) was subjected to silica gel chromatography, eluting with a mixture of chloroform/methanol/water (20/3/0.3), and then concentrated under reduced pressure to obtain 2,3-O-(1,3-propanediyl) ascorbic acid (0.56 g) represented by the following structural formula:

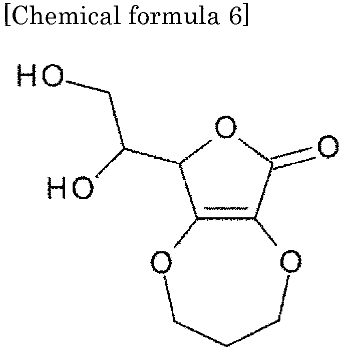

### Synthesis Example 3

### (Synthesis of 2,3-O-(1,4-butanediyl) ascorbic acid)

DMF (35.0 g), ascorbic acid (3.50 g), potassium carbonate (3.0 g), and dibromobutane (4.8 g) were added in an eggplant-shaped flask and stirred at 80°C for 16 hours. After cooling, the mixture was filtered, and the filtrate was concentrated under reduced pressure. The resulting concentrate was dissolved in water. Extraction was performed using isobutyl alcohol, and the organic layer was recovered, to which magnesium sulfate was added. Subsequently, the mixture was filtered, and the filtrate was concentrated under reduced pressure. The resulting residue (3.8 g) was subjected to silica gel chromatography, eluting with a mixture of chloroform/methanol/water (25/3/0.3 to 20/3/0.3), and then concentrated under reduced pressure to obtain 2,3-O-(1,4-butanediyl) ascorbic acid (1.066 g) represented by the following structural formula:

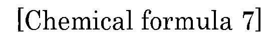

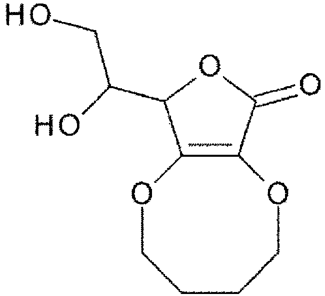

### Synthesis Example 4 and Synthesis Example 5

### (Synthesis of 2,3-O-(2-hydroxypropane-1,3-diyl) ascorbic acid and 2,3-O-(1-hydroxymethyl-1,2-ethanediyl) ascorbic acid)

DMF (13.6 g), water (6.3 g), ascorbic acid (3.5 g), triethylamine (2.0 g), and 2-chloromethyloxirane (2.2 g) were added in an eggplant-shaped flask and stirred at 60°C for 3 hours. After cooling, the filtrate was concentrated under reduced pressure, and the resulting concentrate was dissolved in water. Extraction was performed using isobutyl alcohol, and the organic layer was recovered, to which magnesium sulfate was added. Subsequently, filtration was performed, and the filtrate was concentrated under reduced pressure. The resulting residue (2.4 g) was subjected to silica gel chromatography, eluting with a mixture of chloroform/methanol/water (15/3/0.3 to 10/3/0.3), and concentrated under reduced pressure to obtain a crude product (140 mg). The obtained crude product was separated and purified by HPLC to obtain 2,3-O-(2-hydroxypropane-1,3-diyl) ascorbic acid (58.0 mg) (Synthesis Example 4: represented by the structural formula on the left below) and 2,3-O-(1-hydroxymethyl-1,2-ethanediyl) ascorbic acid (6.6 mg) (Synthesis Example 5: represented by the structural formula on the right below).

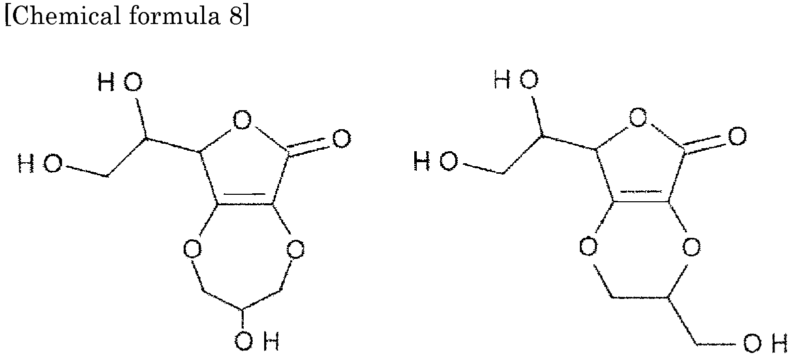

### Synthesis Example 6

### (Synthesis of 2,3-O-(3,3-dimethyleneoxetane) ascorbic acid)

DMF (5.0 g), ascorbic acid (0.528 g), potassium carbonate (0.456 g), and 3,3-bis(bromomethyl)oxetane (0.805 g) were added in an eggplant-shaped flask and stirred at 80°C for 7 hours. After cooling, the mixture was filtered, and the filtrate was concentrated under reduced pressure. The resulting concentrate was dissolved in water. Extraction was performed using isobutyl alcohol, and the organic layer was recovered, to which magnesium sulfate was added. Subsequently, the mixture was filtered, and the filtrate was concentrated under reduced pressure. The resulting residue (3.8 g) was subjected to silica gel chromatography, eluting with a chloroform/methanol/water mixture (20/3/0.3), and then concentrated under reduced pressure to obtain 2,3-O-(3,3-dimethyleneoxetane) ascorbic acid (0.144 g).

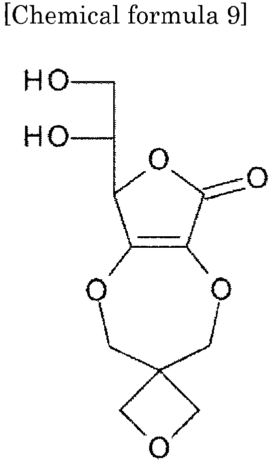

### Synthesis Example 7

### (Synthesis of 2,3-O-(2,2-dimethyl-1,2-ethanediyl) ascorbic acid)

2-O-(2-hydroxyisobutyl) ascorbic acid was synthesized by the method described in Synthesis Example 1 of JP 7267657 B1.

The synthesized 2-O-(2-hydroxyisobutyl) ascorbic acid (0.50 g), THF (5 ml), and p-toluenesulfonic acid monohydrate (0.52 g) were added in an eggplant-shaped flask and stirred at 80°C for 24 hours. After the reaction was completed, ion-exchanged water and isobutanol were added and the mixture was separated. The isobutanol layer was recovered and concentrated under reduced pressure. The resulting residue (0.60 g) was subjected to silica gel chromatography, eluting with a mixture of chloroform/methanol (10/0 to 9.5/0.5) and concentrating under reduced pressure to obtain 2,3-O-(2,2-dimethyl-1,2-ethanediyl) ascorbic acid (37.3 mg).

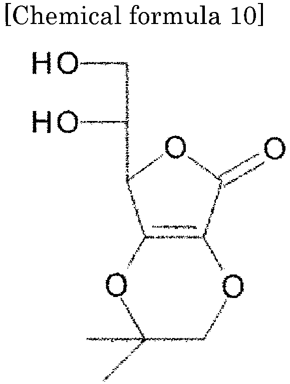

### Synthesis Example 8

### (Synthesis of 2,3-O-(1,1-dimethyl-1,2-ethanediyl) ascorbic acid)

3-O-(2-hydroxyisobutyl) ascorbic acid was synthesized by the method described in Synthesis Example 2 of JP 7267657 B1.

The synthesized 3-O-(2-hydroxyisobutyl) ascorbic acid (4.21 g), THF (25 ml), and p-toluenesulfonic acid monohydrate (4.19 g) were added in an eggplant-shaped flask and stirred at 90°C for 18 hours. After the reaction was completed, ion-exchanged water and ethyl acetate were added and the mixture was separated. The ethyl acetate was recovered and concentrated under reduced pressure. The resulting residue (0.21 g) was subjected to silica gel chromatography, eluting with a mixture of chloroform/methanol (10/0 to 9.5/0.5) and concentrating under reduced pressure to obtain 2,3-O-(1,1-dimethyl-1,2-ethanediyl) ascorbic acid (15.3 mg).

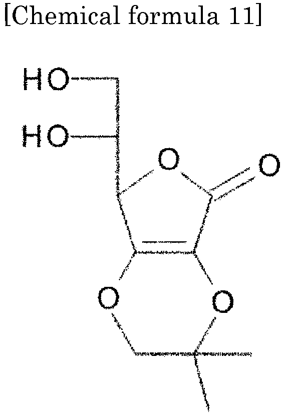

### Synthesis Example 9 and Synthesis Example 10

### (Synthesis of 6-O-butanoyl-2,3-O-(1,4-butanediyl) ascorbic acid and 5,6-O-dibutanoyl-2,3-O-(1,4-butanediyl) ascorbic acid)

In an eggplant-shaped flask were added 2,3-O-(1,4-butanediyl) ascorbic acid (3.0 g) obtained in Synthesis Example 3, N-methylpyrrolidone (30.0 g), and triethylamine (5.1 g), and butanoyl chloride (5.1 g) was added while stirring at 25°C. After stirring for 5 hours at 25°C, the mixture was extracted with ethyl acetate. After washing with water three times, the organic layer was recovered, to which magnesium sulfate was added. Subsequently, the mixture was filtered and concentrated under reduced pressure. The obtained residue (2.5 g) was subjected to silica gel chromatography, eluting with a mixed solvent of hexane/ethyl acetate (4/1 to 1/1), and concentrated under reduced pressure to obtain 6-O-butanoyl-2,3-O-(1,4-butanediyl) ascorbic acid (0.9 g) (Synthesis Example 9: represented by the structural formula on the left below) and 5,6-O-dibutanoyl-2,3-O-(1,4-butanediyl) ascorbic acid (1.4 g) (Synthesis Example 10: represented by the structural formula on the right below).

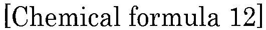

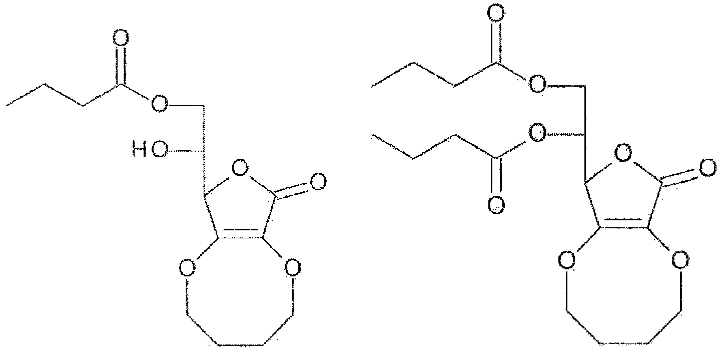

### Synthesis Example 11 and Synthesis Example 12

### (Synthesis of 6-O-octanoyl-2,3-O-(1,4-butanediyl) ascorbic acid and 5,6-O-dioctanoyl-2,3-O-(1,4-butanediyl) ascorbic acid)

In an eggplant-shaped flask were added 2,3-O-(1,4-butanediyl) ascorbic acid (3.0 g) obtained in Synthesis Example 3, N-methylpyrrolidone (30.0 g), and triethylamine (3.4 g), and then octanoyl chloride (5.0 g) was added while stirring at 25°C. The mixture was stirred at 25°C for 2 hours and extracted with ethyl acetate. The mixture was washed with water three times, and the organic layer was recovered, to which magnesium sulfate was added. The mixture was then filtered and concentrated under reduced pressure. The obtained residue (5.1 g) was subjected to silica gel chromatography, eluting with a mixed solvent of hexane/ethyl acetate (5/1 to 1/1), and concentrated under reduced pressure to obtain 6-O-octanoyl-2,3-O-(1,4-butanediyl) ascorbic acid (2.4 g) (Synthesis Example 11: represented by the structural formula on the left below) and 5,6-O-dioctanoyl-2,3-O-(1,4-butanediyl)ascorbic acid (1.6 g) (Synthesis Example 12: represented by the structural formula on the right below), which are represented in the following structural formulas.

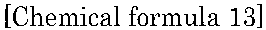

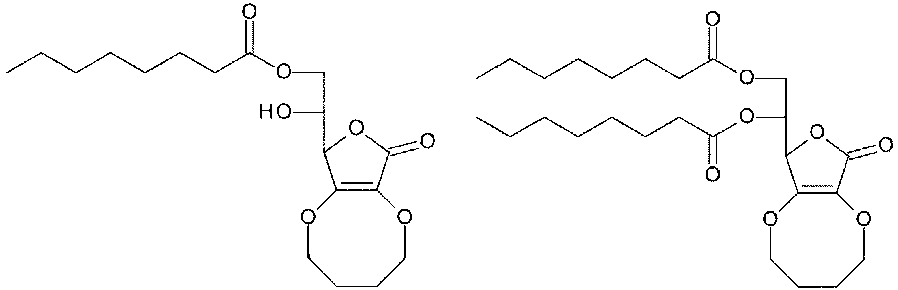

### Synthesis Example 13

### (Synthesis of 6-O-lauroyl-2,3-O-(1,4-butanediyl) ascorbic acid)

In an eggplant-shaped flask were added 2,3-O-(1,4-butanediyl) ascorbic acid (3.0 g) obtained in Synthesis Example 3, N-methylpyrrolidone (30.0 g), and triethylamine (1.7 g), and then dodecanoyl chloride (3.4 g) was added while stirring at 25°C. The mixture was stirred at 25°C for 4 hours and extracted with ethyl acetate. The mixture was washed with water three times, and the organic layer was recovered, to which magnesium sulfate was added. The mixture was then filtered and concentrated under reduced pressure. The resulting residue (6.1 g) was subjected to silica gel chromatography, eluted with a mixed solvent of hexane/ethyl acetate (8/3 to 1/1), and concentrated under reduced pressure to obtain 6-O-lauroyl-2,3-O-(1,4-butanediyl) ascorbic acid (3.1 g) represented by the following structural formula.

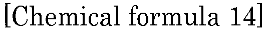

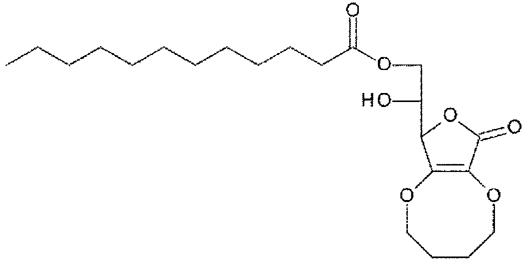

### Synthesis Example 14

### (Synthesis of 5,6-O-dilauroyl-2,3-O-(1,4-butanediyl) ascorbic acid)

In an eggplant-shaped flask were added 6-O-lauroyl-2,3-O-(1,4-butanediyl) ascorbic acid (1.5 g) obtained in Synthesis Example 13, 4-dimethylaminopyridine (DMAP: 19.0 mg), and dodecanoyl chloride (1.2 g), followed by stirring for 4 hours at 25° C. The resulting residue (3.2 g) was subjected to silica gel chromatography, eluted with a mixed solvent of hexane /ethyl acetate (4/1), and concentrated under reduced pressure to obtain 5,6-O-dilauroyl -2,3-O- (1,4-butanediyl) ascorbic acid (0.9 g) represented by the following structural formula.

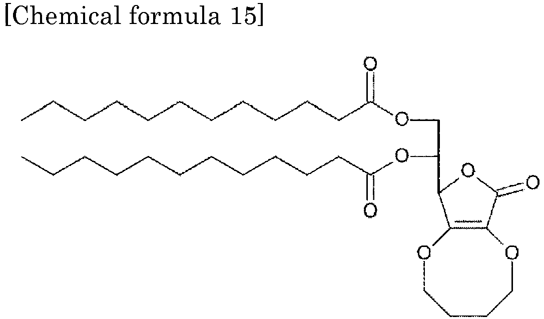

### Synthesis Example 15

### (Synthesis of 6-O-palmitoyl-2,3-O-(1,4-butanediyl) ascorbic acid)

In an eggplant-shaped flask were added 2,3-O-(1,4-butanediyl) ascorbic acid (2.0 g) obtained in Synthesis Example 3, N-methylpyrrolidone (30.0 g), and triethylamine (1.1 g), and hexadecanoyl chloride (2.6 g) was added while stirring at 0°C. After stirring for 5 hours at 25°C, the mixture was extracted with ethyl acetate. The mixture was washed twice with water, and the organic layer was recovered, to which magnesium sulfate was added. Subsequently, the mixture was filtered and concentrated under reduced pressure. The resulting residue (4.7 g) was subjected to silica gel chromatography, eluting with a mixed solvent of hexane/ethyl acetate (5/1 to 1/1), and concentrated under reduced pressure to obtain 6-O-palmitoyl-2,3-O-(1,4-butanediyl) ascorbic acid (1.3 g) represented by the following structural formula.

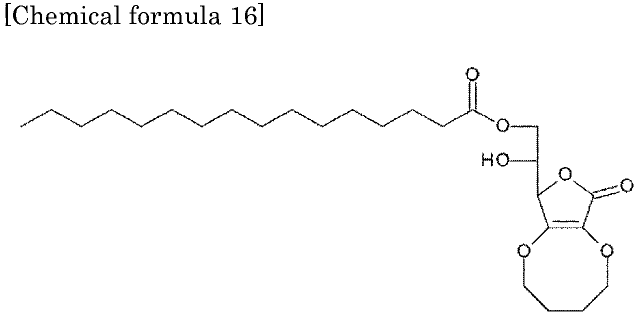

### Synthesis Example 16

### (Synthesis of 5,6-O-dipalmitoyl-2,3-O-(1,4-butanediyl) ascorbic acid)

In an eggplant-shaped flask were added 6-O-palmitoyl-2,3-O-(1,4-butanediyl) ascorbic acid (1.6 g) obtained in Synthesis Example 15, DMAP (22.0 mg), and hexadecanoyl chloride (1.8 g), followed by stirring at 60°C for 4 hours and then at 25°C for 16 hours. The mixture was then extracted with a mixed solvent of hexane/ethyl acetate (1/1), and the organic layer was recovered, to which magnesium sulfate was added. The mixture was then filtered and concentrated under reduced pressure. The resulting residue (3.3 g) was subjected to silica gel chromatography, eluting with a mixed solvent of hexane/ethyl acetate (4/1) and then concentrating under reduced pressure to obtain 5,6-O-dipalmitoyl-2,3-O-(1,4-butanediyl) ascorbic acid (0.9 g) represented by the following structural formula.

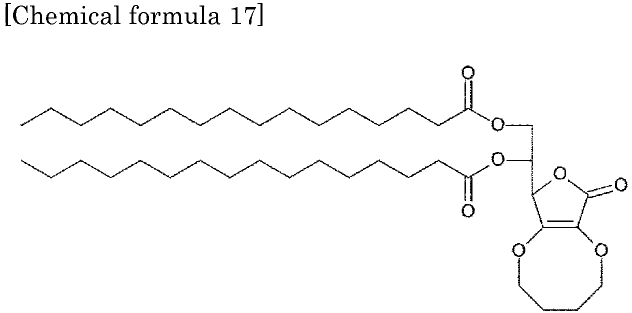

### Synthesis Example 17 and Synthesis Example 18

### (Synthesis of 6-O-isostearoyl-2,3-O-(1,4-butanediyl) ascorbic acid and 5,6-O-diisostearoyl-2,3-O-(1,4-butanediyl) ascorbic acid)

In an eggplant-shaped flask were added 2,3-O-(1,4-butanediyl) ascorbic acid (3.0 g) obtained in Synthesis Example 3, N-methylpyrrolidone (30.0 g), and triethylamine (3.4 g), and then isostearoyl chloride (9.4 g) was added while stirring at 25°C. After stirring for 3 hours at 25°C, the mixture was extracted with ethyl acetate. After washing with water twice, the organic layer was recovered, to which magnesium sulfate was added. Subsequently, the mixture was filtered and concentrated under reduced pressure. The obtained residue (9.8 g) was subjected to silica gel chromatography, eluting with a mixed solvent of hexane/ethyl acetate (4/1 to 1/1), and concentrated under reduced pressure to obtain 6-O-isostearoyl-2,3-O-(1,4-butanediyl) ascorbic acid (2.5 g) (Synthesis Example 17: represented by the upper structural formula below) and 5,6-O-diisostearoyl-2,3-O-(1,4-butanediyl) ascorbic acid (1.8 g) (Synthesis Example 18: represented by the lower structural formula below).

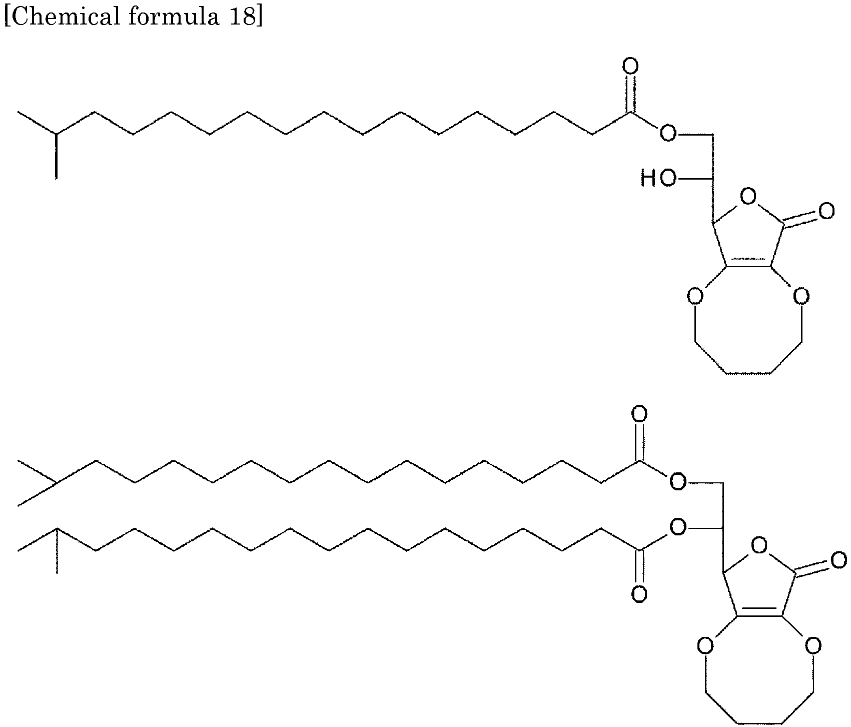

### Synthesis Example 19

### (Synthesis of 5-O-butanoyl-6-O-octanoyl-2,3-O-(1,4-butanediyl) ascorbic acid)

In an eggplant-shaped flask were added 6-O-octanoyl-2,3-O-(1,4-butanediyl) ascorbic acid (1.0 g) obtained in Synthesis Example 11, acetonitrile (5.0 g), and triethylamine (0.6 g), and then butanoyl chloride (0.5 g) was added while stirring at 25°C. The mixture was stirred at 25°C for 2 hours and extracted with ethyl acetate. The mixture was washed twice with water, and the organic layer was recovered, to which magnesium sulfate was added. Subsequently, the mixture was filtered and concentrated under reduced pressure. The resulting residue (1.5 g) was subjected to silica gel chromatography, eluting with a mixed solvent of hexane/ethyl acetate (4/1 to 3/1), and concentrated under reduced pressure to obtain 5-O-butanoyl-6-O-octanoyl-2,3-(1,4-butanediyl) ascorbic acid (0.8 g) represented by the following structural formula.

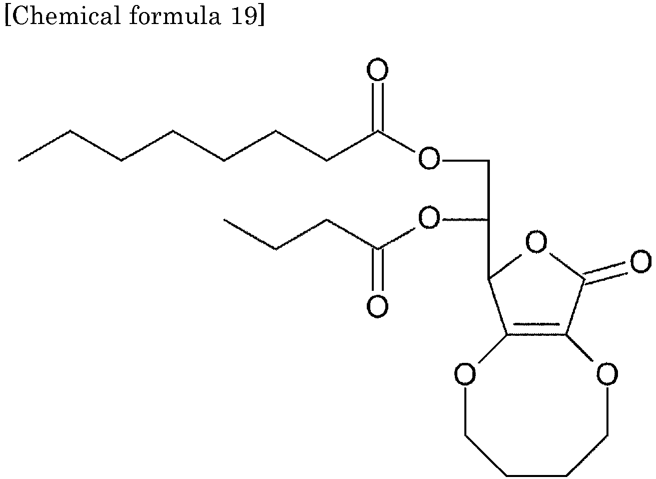

### Synthesis Example 20 and Synthesis Example 21

### (Synthesis of 6-O-butanoyl-2,3-O-(1,3 -propanediyl) ascorbic acid and 5,6-O-dibutanoyl-2,3-O-(1,3- propanediyl) ascorbic acid)

In an eggplant-shaped flask were added 2,3-O-(1,3- propanediyl) ascorbic acid (2.2 g) obtained in Synthesis Example 2, N-methylpyrrolidone (22.0 g), and triethylamine (5.0 g), and then butanoyl chloride (2.6 g) was added while stirring at 25°C. The mixture was stirred at 25°C for 2 hours and extracted with ethyl acetate. The mixture was washed with water four times, and the organic layer was recovered, to which magnesium sulfate was added. Subsequently, the mixture was filtered and concentrated under reduced pressure. The resulting residue (4.2 g) was subjected to silica gel chromatography, eluting with a mixed solvent of hexane/ethyl acetate (3/1 to 2/3), and concentrated under reduced pressure to obtain 6-O-butanoyl-2,3-O-(1,3- propanediyl) ascorbic acid (0.7 g) (Synthesis Example 20: represented by the structural formula on the left below) and 5,6-O-dibutanoyl-2,3-O-(1,3 - propanediyl) ascorbic acid (1.9 g) (Synthesis Example 21: represented by the structural formula on the right below).

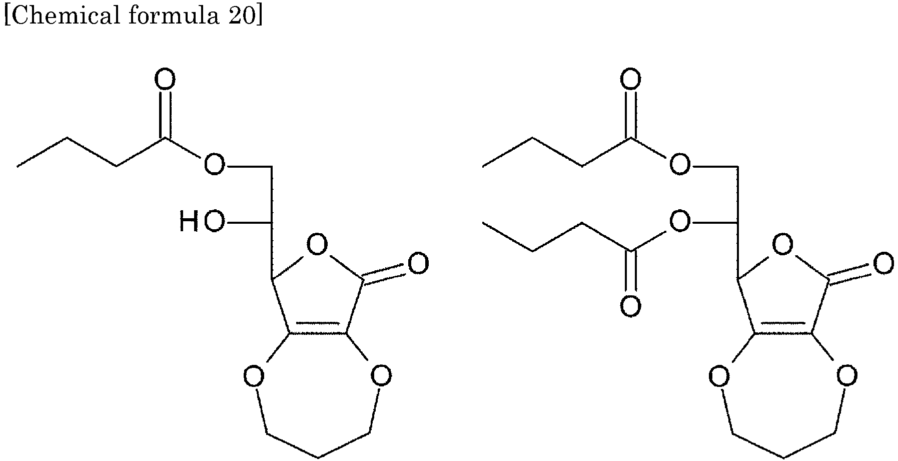

### Synthesis Example 22 and Synthesis Example 23

### (Synthesis of 6-O-octanoyl-2,3-O-(1,3 -propanediyl) ascorbic acid and 5,6-O-dioctanoyl-2,3-O-(1,3- propanediyl) ascorbic acid)

In an eggplant-shaped flask were added 2,3-O-(1,3- propanediyl) ascorbic acid (2.2 g) obtained in Synthesis Example 2, N-methylpyrrolidone (22.0 g), and triethylamine (5.0 g), and then octanoyl chloride (6.0 g) was added while stirring at 25°C. The mixture was stirred at 25°C for 3 hours and extracted with ethyl acetate. The mixture was washed with water four times, and the organic layer was recovered, to which magnesium sulfate was added. Subsequently, the mixture was filtered and concentrated under reduced pressure. The resulting residue (7.6 g) was subjected to silica gel chromatography, eluting with a mixed solvent of hexane/ethyl acetate (4/1 to 1/1), and concentrated under reduced pressure to obtain 6-O-octanoyl-2,3-O-(1,3- propanediyl) ascorbic acid (1.3 g) (Synthesis Example 22: represented by the structural formula on the left below) and 5,6-O-dioctanoyl-2,3-O-(1,3-propanediyl) ascorbic acid (2.3 g) (Synthesis Example 23: represented by the structural formula on the right below).

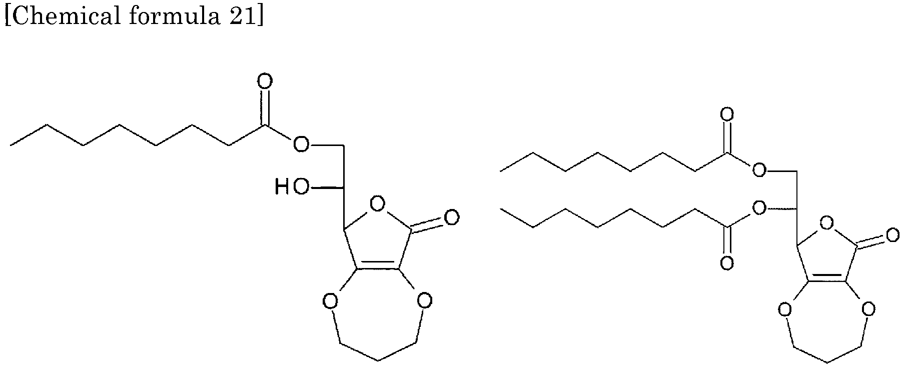

### Synthesis Example 24 and Synthesis Example 25

### (Synthesis of 6-O-lauroyl-2,3-O-(1,3 -propanediyl) ascorbic acid and 5,6-O-dilauroyl-2,3-O-(1,3- propanediyl) ascorbic acid)

In an eggplant-shaped flask were added 2,3-O-(1,3- propanediyl) ascorbic acid (2.2 g) obtained in Synthesis Example 2, N-methylpyrrolidone (22.0 g), and triethylamine (11.0 g), and then dodecanoyl chloride (7.8 g) was added while stirring at room temperature. The mixture was stirred at 25°C for 3 hours and extracted with ethyl acetate. The mixture was washed with water three times, and the organic layer was recovered, to which magnesium sulfate was added. Subsequently, the mixture was filtered and concentrated under reduced pressure. The resulting residue (6.6 g) was subjected to silica gel chromatography, eluting with a mixed solvent of hexane/ethyl acetate (4/1 to 1/1), and concentrated under reduced pressure to obtain 6-O-lauroyl-2,3-O-(1,3- propanediyl) ascorbic acid (1.3 g) (Synthesis Example 24: represented by the upper structural formula below) and 5,6-O-dilauroyl-2,3-O-(1,3-propanediyl) ascorbic acid (2.3 g) (Synthesis Example 25: represented by the lower structural formula below).

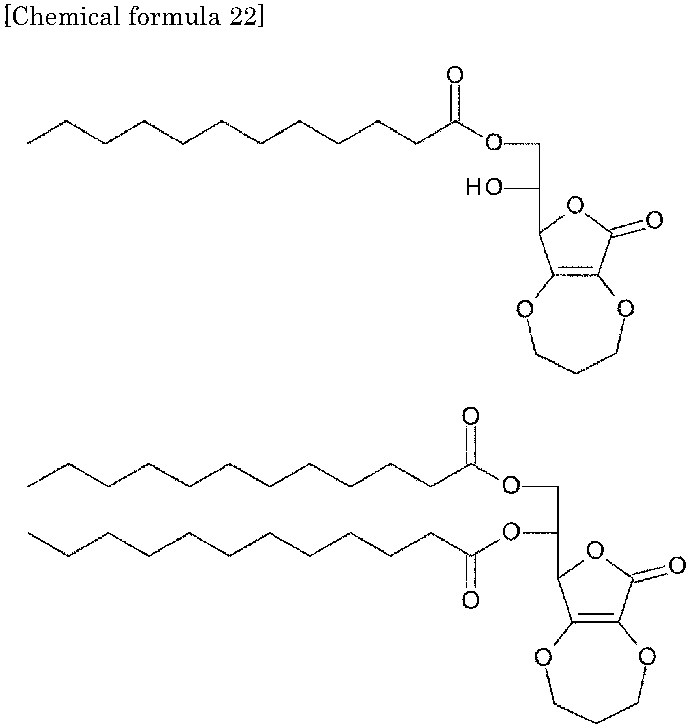

### Synthesis Example 26 and Synthesis Example 27

### (Synthesis of 6-O-palmitoyl-2,3-O-(1,3 -propanediyl) ascorbic acid and 5,6-O-dipalmitoyl-2,3-O-(1,3- propanediyl) ascorbic acid)

In an eggplant-shaped flask were added 2,3-O- (1,3- propanediyl) ascorbic acid (2.2 g) obtained in Synthesis Example 2, N-methylpyrrolidone (22.0 g), and triethylamine (3.9 g), and then hexadecanoyl chloride (9.9 g) was added while stirring at 25°C. The mixture was stirred at 25°C for 3 hours and extracted with ethyl acetate. The mixture was washed twice with water, and the organic layer was recovered, to which magnesium sulfate was added. Subsequently, the mixture was filtered and concentrated under reduced pressure. The obtained residue (13.4 g) was subjected to silica gel chromatography, eluting with a mixed solvent of hexane/ethyl acetate (6/1 to 1/1), and concentrated under reduced pressure to obtain 6-O-palmitoyl-2,3-O-(1,3- propanediyl) ascorbic acid (1.3 g) (Synthesis Example 26: represented by the upper structural formula below) and 5,6-O-dipalmitoyl-2,3-O-(1,3-propanediyl) ascorbic acid (2.6 g) (Synthesis Example 27: represented by the lower structural formula below).

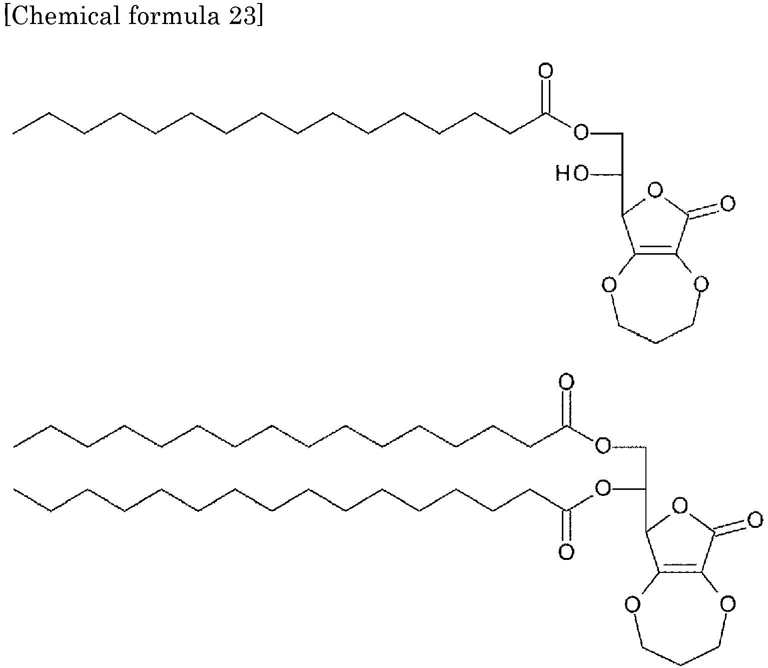

### Synthesis Example 28

### (Synthesis of 6-O-isostearoyl-2,3-O-(1,3- propanediyl) ascorbic acid)

In an eggplant-shaped flask were added 2,3-O-(1,3- propanediyl) ascorbic acid (3.0 g) obtained in Synthesis Example 2, N-methylpyrrolidone (30.0 g), and triethylamine (1.8 g), and then isostearoyl chloride (5.0 g) was added with stirring. The mixture was stirred at 25°C for 4 hours and extracted with ethyl acetate. The mixture was washed with water three times, and the organic layer was recovered, to which magnesium sulfate was added. Subsequently, the mixture was filtered and concentrated under reduced pressure. The resulting residue (8.6 g) was subjected to silica gel chromatography, eluting with a mixed solvent of hexane/ethyl acetate (4/1 to 1/1), and concentrated under reduced pressure to obtain 6-O-isostearoyl-2,3-0-(1,3- propanediyl) ascorbic acid (2.4 g) represented by the following structural formula.

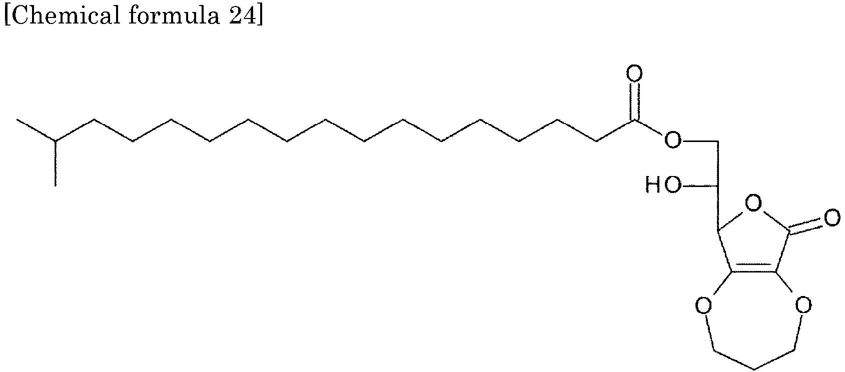

### Synthesis Example 29

### (Synthesis of 5,6-O-diisostearoyl-2,3-O-(1,3-propanediyl) ascorbic acid)

In an eggplant-shaped flask were added 6-isostearoyl-2,3-O-(1,3-propanediyl) ascorbic acid (1.4 g) obtained in Synthesis Example 28, DMAP (22.0 mg), and isostearoyl chloride (1.4 g). N-methylpyrrolidone (14.0 g) was then added, and the mixture was stirred at 25°C for 7 hours and concentrated under reduced pressure. The resulting residue (3.8 g) was subjected to silica gel chromatography, eluting with a mixed solvent of hexane/ethyl acetate (4/1), and concentrated under reduced pressure to obtain 5,6-O-diisostearoyl-2,3-O-(1,3-propanediyl) ascorbic acid (0.17 g) represented by the following structural formula.

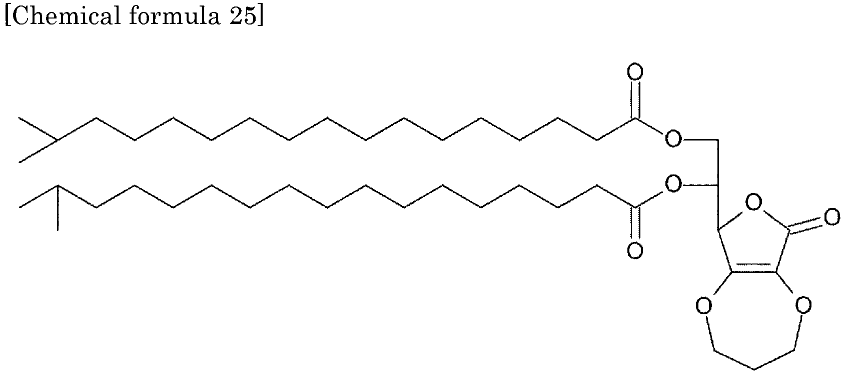

### Synthesis Example 30

### (Synthesis of 5-butanoyl-6-O-palmitoyl-2,3-O-(1,3- propanediyl) ascorbic acid)

In an eggplant-shaped flask were added 6-O-palmitoyl-2,3-O-(1,3-propanediyl) ascorbic acid (0.8 g) obtained in Synthesis Example 26, acetonitrile (4.0 g), and triethylamine (0.36 g), and then butanoyl chloride (0.3 g) was added while stirring at 25°C. The mixture was stirred at 25°C for 2 hours and extracted with ethyl acetate. The mixture was washed twice with water, and the organic layer was recovered, to which magnesium sulfate was added. Subsequently, the mixture was filtered and concentrated under reduced pressure. The resulting residue (0.9 g) was subjected to silica gel chromatography, eluting with a mixed solvent of hexane/ethyl acetate (4/1 to 3/1), and concentrated under reduced pressure to obtain 5-O-butanoyl-6-O-palmitoyl-2,3-O-(1,3-propanediyl) ascorbic acid (0.6 g) represented by the following structural formula.

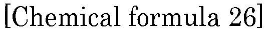

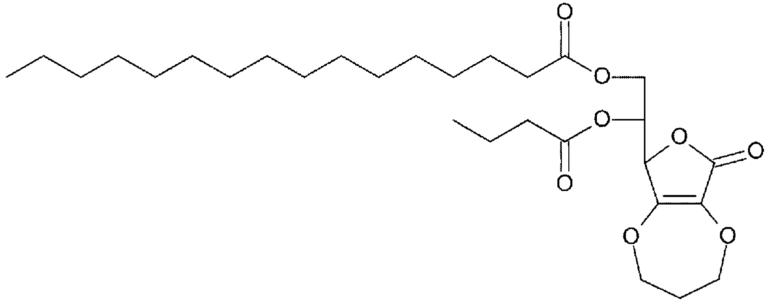

### Synthesis Example 31 and Synthesis Example 32

### (Synthesis of 6-O-isostearoyl-2,3-O-(1,2- ethanediyl) ascorbic acid and 5,6-O-diisostearoyl-2,3-O-(1,2-ethanediyl) ascorbic acid)

In an eggplant-shaped flask were added 2,3-O-(1,2-ethanediyl) ascorbic acid (1.9 g) obtained in Synthesis Example 1, N-methylpyrrolidone (20.0 g), and triethylamine (4.8 g), and isostearoyl chloride (6.8 g) was added while stirring at 25°C. The mixture was stirred at 25°C for 3 hours and extracted with ethyl acetate. The mixture was washed with water four times, and the organic layer was recovered, to which magnesium sulfate was added. Subsequently, the mixture was filtered and concentrated under reduced pressure. The obtained residue (9.2 g) was subjected to silica gel chromatography, eluting with a mixed solvent of hexane/ethyl acetate (4/1 to 1/1), and concentrated under reduced pressure to obtain 6-O-isostearoyl-2,3-O-(1,2-ethanediyl) ascorbic acid (1.0 g) (Synthesis Example 31: represented by the upper structural formula below) and 5,6-O-diisostearoyl-2,3-O-(1,2-ethanediyl) ascorbic acid (1.0 g) (Synthesis Example 32: represented by the lower structural formula below).

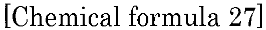

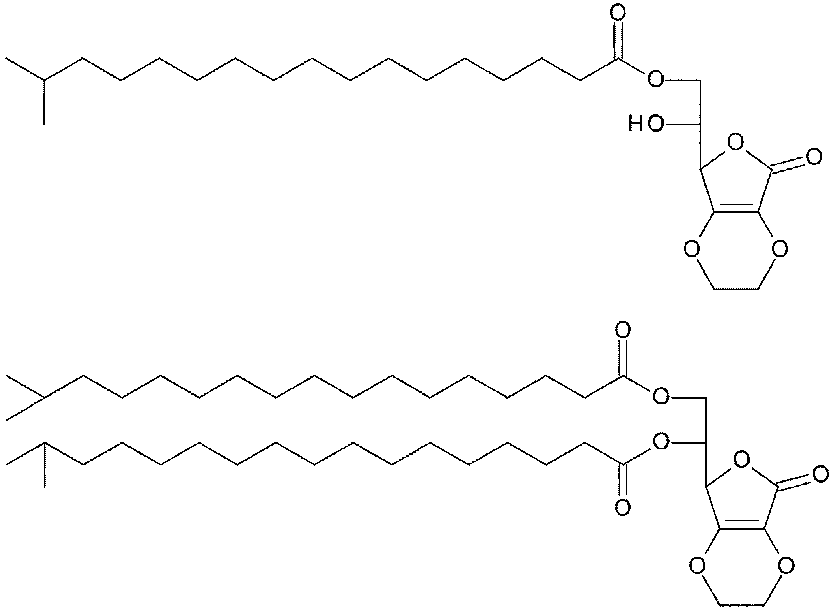

### Synthesis Example 33

### (Synthesis of 4-hydroxy-2,6,9,14-tetraoxatricyclo [6.6.0.0^{1.5}] tetradecan-7-one)

In an eggplant-shaped flask, were added 2,3-O-(1,4-butanediyl) ascorbic acid (1.3 g) obtained in Synthesis Example 3, water (8.0 g), and isopropyl alcohol (2.0 g), and while stirring at 25°C, 10% aqueous sodium hydroxide solution (4.5 g) was added, followed by stirring at 25°C for 30 minutes. Subsequently, 17% aqueous hydrochloric acid solution (2.4 g) was added, and the mixture was allowed to stand at 25°C for 16 hours, followed by extraction with ethyl acetate. The mixture was washed with water three times, and the organic layer was recovered, to which magnesium sulfate was added. Subsequently, the mixture was filtered and concentrated under reduced pressure. The resulting residue (0.4 g) was subjected to silica gel chromatography, eluted with a mixed solvent of hexane/ethyl acetate (3/1 to 1/1), and concentrated under reduced pressure to obtain 4-hydroxy-2,6,9,14-tetraoxatricyclo [6.6.0.0^{1.5}] tetradecan-7-one (125 mg) represented by the following structural formula.

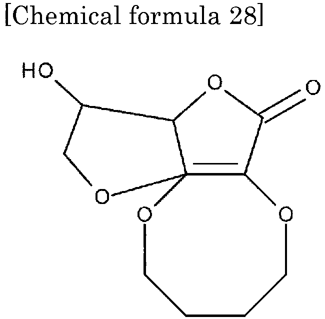

### Example 34

### (Synthesis of 4-hydroxy-2,6,9,13-tetraoxatricyclo [6.5.0.0^{1.5}] tridecan-7-one)

In an eggplant-shaped flask, were added 2,3-O-(1,3-propanediyl) ascorbic acid (2.5 g) obtained in Synthesis Example 2, water (16.0 g), and isopropyl alcohol (4.0 g). While stirring at 25°C, 10% aqueous sodium hydroxide solution (9.0 g) was added, followed by stirring at 25°C for 8 hours. Then, 17% aqueous hydrochloric acid solution (7.5 g) was then added, and the mixture was allowed to stand at 25°C for 16 hours. Ethyl acetate was then added for extraction. The mixture was washed three times with water, and the organic layer was recovered, to which magnesium sulfate was added. Subsequently, the mixture was filtered and concentrated under reduced pressure. The resulting residue (1.7 g) was subjected to silica gel chromatography, eluted with a mixed solvent of hexane/ethyl acetate (1/1), and concentrated under reduced pressure to obtain 4-hydroxy-2,6,9,13-tetraoxatricyclo [6.5.0.0^{1.5}] tridecan-7-one (464 mg) represented by the following structural formula.

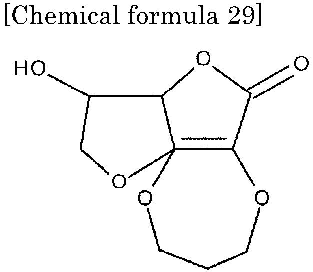

### Synthesis Example 35

### (Synthesis of 5,6-O-isopropylidene-2,3-O-(1,4-butanediyl) ascorbic acid)

In an eggplant-shaped flask, 2,3-O-(1,4-butanediyl) ascorbic acid (920 mg) obtained in Synthesis Example 3, acetonitrile (9.0 mL), 2,2-dimethoxypropane (824 mg), and p-toluenesulfonic acid monohydrate (76 mg) were added in this order, and the mixture was stirred at 25°C for 2 hours. The mixture was neutralized with triethylamine and concentrated under reduced pressure. Ethyl acetate was added to the resulting concentrate for extraction. The mixture was washed once with water and twice with saturated brine, and magnesium sulfate was added to the recovered organic layer. The mixture was then filtered and concentrated under reduced pressure. The resulting residue (1.1 g) was dissolved in a mixed solvent of hexane/ethyl acetate (5/3) with heating, allowed to cool, and then allowed to stand overnight at room temperature. The precipitated solid was collected by filtration, washed with hexane, and vacuum dried to obtain 5,6-O-isopropylidene-2,3-O-(1,4-butanediyl) ascorbic acid (619 mg) represented by the following structural formula.

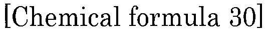

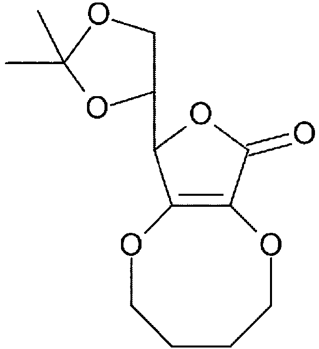

### Synthesis Example 36

### (Synthesis of 5,6-O-isopropylidene-2,3-O-(1,3-propanediyl) ascorbic acid)

In an eggplant-shaped flask, 2,3-O-(1,3-propanediyl) ascorbic acid (650 mg) obtained in Synthesis Example 2, acetone (6.0 mL), 2,2-dimethoxypropane (620 mg), and p-toluenesulfonic acid monohydrate (57 mg) were added in this order, and the mixture was stirred at 25°C for 2.5 hours. The mixture was neutralized with aqueous sodium hydroxide and concentrated under reduced pressure, and ethyl acetate was added to the resulting concentrate for extraction. The mixture was washed once with water and twice with saturated brine, and magnesium sulfate was added to the recovered organic layer. The mixture was then filtered and concentrated under reduced pressure. The resulting residue (750 mg) was dissolved in a mixture of isopropanol and methanol (3/2) with heating, allowed to cool, and then allowed to stand overnight at room temperature. The precipitated solid was collected by filtration, washed with isopropanol, and vacuum dried to obtain 5,6-O-isopropylidene-2,3-O-(1,3-propanediyl) ascorbic acid (303 mg) represented by the following structural formula.

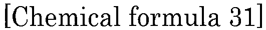

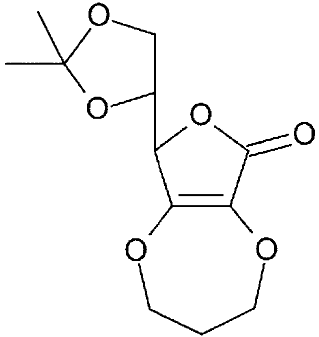

### Synthesis Example 37

### (Synthesis of 5,6-O-(phenylmethylene)-2,3-O-(1,4-butanediyl) ascorbic acid)

In an eggplant-shaped flask, 2,3-O-(1,4-butanediyl) ascorbic acid (920 mg) obtained in Synthesis Example 3, acetonitrile (9.0 mL), α,α-dimethoxytoluene (669 mg), and p-toluenesulfonic acid monohydrate (76 mg), were added in this order, and the mixture was stirred at 25°C for 19 hours. The mixture was neutralized with triethylamine and concentrated under reduced pressure. Water and ethyl acetate were added in this order to the resulting concentrate, and the concentrate was washed. The solid was recovered by filtration and vacuum dried to obtain 5,6-O-(phenylmethylene)-2,3-O-(1,4-butanediyl) ascorbic acid (226 mg) represented by the following structural formula.

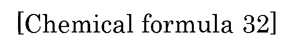

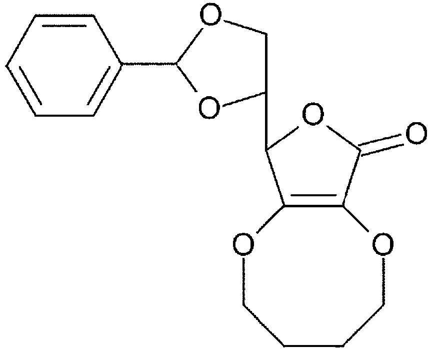

### Synthesis Example 38

### (Synthesis of 5,6-O-(phenylmethylene)-2,3-O-(1,3-propanediyl) ascorbic acid)

In an eggplant-shaped flask, 2,3-O-(1,3-propanediyl) ascorbic acid (650 mg) obtained in Synthesis Example 2, acetonitrile (6.0 mL), α,α-dimethoxytoluene (502 mg), and p-toluenesulfonic acid monohydrate (57 mg) were added in this order, and the mixture was stirred at 25°C for 5 hours. The mixture was neutralized with triethylamine and concentrated under reduced pressure. The concentrate was washed with water and then with a mixure of hexane/ethyl acetate (5/1). The solid was collected by filtration and vacuum dried to obtain 5,6-0-(phenylmethylene)-2,3-0-(1,3-propanediyl) ascorbic acid (303 mg) represented by the following structural formula.

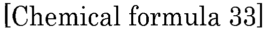

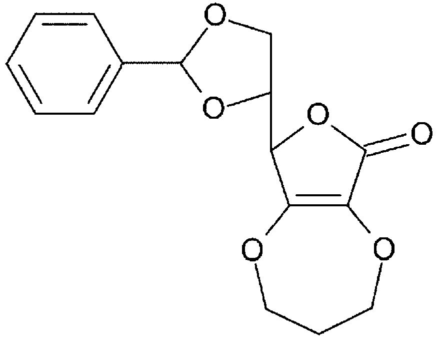

### Synthesis Example 39

### (Synthesis of 6-O-benzyl-2,3-O-(1,4-butanediyl) ascorbic acid)

In an eggplant-shaped flask were added 2,3-O-(1,4-butanediyl) ascorbic acid (318 mg) obtained in Synthesis Example 3 and DMF (5.0 mL). Being cooled in an ice bath, 2-picoline borane (1,000 mg) and trimethylsilyl chloride (543 mg) were added, and then the mixture was stirred in a 50°C water bath for 1 hour. Ice water was added, followed by stirring for an additional 1 hour. Ethyl acetate (10 mL) was added and the mixture was separated. The organic layer was recovered, and the aqueous layer was extracted with ethyl acetate (50 mL). The recovered organic layer was washed twice with saturated brine (50 mL). Magnesium sulfate was added, followed by filtration and concentration under reduced pressure. The resulting residue (750 mg) was subjected to silica gel chromatography, eluting with a mixed solvent of hexane/ethyl acetate (1/1 to 1/2), and concentrated under reduced pressure to obtain 6-O-benzyl-2,3-O-(1,4-butanediyl) ascorbic acid (90 mg).

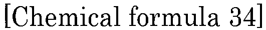

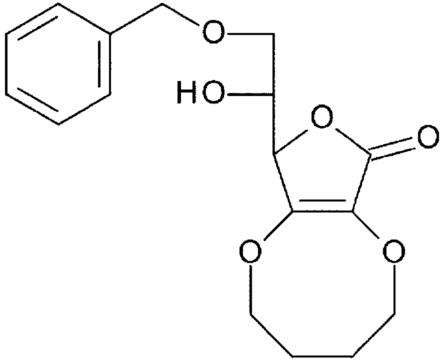

The products obtained in Synthesis Examples 1 to 39 were subjected to mass spectrometry using an LCMS-2020 (manufactured by Shimadzu Corporation). The measurement results are shown in Tables 1 and 2.

**[Table 1]**

| Synthesis Example No. | Ionization | Measured value |
|---|---|---|
| 1 | [M+HCOO]⁻ | 247 |
| 2 | [M+HCOO]⁻ | 261 |
| 3 | [M+HCOO]⁻ | 275 |
| 4 | [M+HCOO]⁻ | 277 |
| 5 | [M+HCOO]⁻ | 277 |
| 6 | [M+HCOO]⁻ | 303 |
| 7 | [M+H]⁺ | 230 |
| 8 | [M+H]⁺ | 230 |
| 9 | [M+H]⁺ | 301 |
| 10 | [M+H]⁺ | 371 |
| 11 | [M+H]⁺ | 357 |
| 12 | [M+H]⁺ | 483 |
| 13 | [M+H]⁺ | 413 |
| 14 | [M+H]⁺ | 595 |
| 15 | [M+H]⁺ | 469 |
| 16 | [M+H]⁺ | 708 |
| 17 | [M+H]⁺ | 497 |

| | | |
|---|---|---|
| *Ionization was performed by electrospray ionization (ESI). | | |

**[Table 2]**

| Synthesis Example No. | Ionization | Measured value |
|---|---|---|
| 18 | [M+H]⁺ | 764 |
| 19 | [M+H]⁺ | 427 |
| 20 | [M+H]⁺ | 287 |
| 21 | [M+H]⁺ | 357 |
| 22 | [M+H]⁺ | 343 |
| 23 | [M+H]⁺ | 469 |
| 24 | [M+H]⁺ | 399 |
| 25 | [M+H]⁺ | 581 |
| 26 | [M+H]⁺ | 455 |
| 27 | [M+H]⁺ | 693 |
| 28 | [M+H]⁺ | 483 |
| 29 | [M+H]⁺ | 749 |
| 30 | [M+H]⁺ | 525 |
| 31 | [M+NH₄]⁺ | 486 |
| 32 | [M+NH₄]⁺ | 752 |
| 33 | [M+H]⁺ | 231 |
| 34 | [M+H]⁺ | 234 |
| 35 | [M+H]⁺ | 271 |
| 36 | [M+H]⁺ | 257 |
| 37 | [M+H]⁺ | 319 |
| 38 | [M+H]⁺ | 305 |
| 39 | [M+H]⁺ | 321 |

| | | |
|---|---|---|
| *Ionization was performed by electrospray ionization (ESI). | | |

The products obtained in Synthesis Examples 1 to 39 were subjected to ¹ H-NMR using a JNM-ECS400 (manufactured by JEOL Ltd.). The measurement results are shown in Tables 3 to 6.

**[Table 3]**

| Synthesis Ex. No. | Measurement condition | Chemical Shift δ (ppm) |
|---|---|---|
| 1 | 400MHz, CD₃OD | 3.67(2H, d), 3.84(1H, dt), 4.24-4.26(2H, m), 4.40-4.43(2H, m), 5.06(1H, d) |
| 2 | 400MHz, CD₃OD | 2.21(2H, quin), 3.65(1H, d), 3.65(1H, d), 3.87(1H, dt), 4.14-4.18(2H, m), 4.34-4.38(2H, m), 4.87(1H, d) |
| 3 | 400MHz, CD₃OD | 1.74-1.83(1H, m), 1.95-2.17(3H, m), 3.66(2H, d), 3.88(1H, dt), 4.04-4.15(2H, m), 4.53-4.58(1H, m), 4.66-4.73(1H, m),4.94(1H, d) |
| 4 | 400MHz, CD₃OD | 3.66(2H, d), 3.90(1H, dt), 4.08(1H, d), 4.12-4.19(2H, m), 4.10-4.13(1H, m), 4.36 (1H, dd), 4.87(1H, d) |
| 5 | 400MHz, CD₃OD | 3.67(2H, d), 3.79(2H, d), 3.85(1H, dt), 4.14-4.18(1H, m), 4.24(1H, dd), 4.54(1H, dd) |
| 6 | 400MHz, CD₃OD | 3.64(1H, d), 3.64(1H, d), 3.86(1H, dt), 4.36(1H, d), 4.42(1H, d), 4.47-4.53(4H, m), 4.57(1H, d), 4.63(1H, d), 5.06(1H, d) |
| 7 | 400MHz, CDCl₃ | 1.35(3H, s), 1.37(3H, s), 3.77(1H, d), 3.91(1H, d), 4.00(2H, d), 4.34(1H, br), 4.44(1H, br) |
| 8 | 400MHz, CDCl₃ | 1.36(6H, s), 3.84(2H, d), 3.95(1H, m), 4.00(2H, m), 4.97(1H, d) |
| 9 | 400MHz, CDCl₃ | 0.96(3H, t), 1.67(2H, m), 1.77(1H, m), 2.02(2H, m), 2.13(1H, m), 2.35(2H, t), 4.14(3H, m), 4.26(1H, dd), 4.39(1H, dd), 4.52(1H, m), 4.72(1H, m), 4.77(1H, d) |
| 10 | 400MHz, CDCl₃ | 0.95(6H, t), 1.64(4H, m), 1.81(2H, m), 2.03(2H, m), 2.30(4H, m), 4.14(2H, m), 4.27(1H, dd), 4.40(1H, dd), 4.50(1H, m), 4.57(1H, m),4.89(1H, d), 5.42(1H, dt) |
| 11 | 400MHz, CDCl₃ | 0.88(3H, t), 1.29(8H, brs), 1.63(2H, quin), 1.77(1H, m), 2.02(2H, m), 2.13(1H, m), 2.36(2H, t), 4.14(3H, m), 4.26(1H, dd), 4.38(1H, dd), 4.52(1H, dt), 4.72(1H, m), 4.77(1H, d) |
| 12 | 400MHz, CDCl₃ | 0.88(6H, t), 1.28(16H, brs), 1.60(4H, m), 1.80(2H, m), 2.03(2H, m), 2.30(4H, m), 4.14(2H, dt), 4.26(1H, dd), 4.39(1H, dd), 4.48(1H, ddd), 4.57(1H, ddd), 4.88(1H, d), 5.41(1H, ddd) |

**[Table 4]**

| Synthesis Ex. No. | Measurement condition | Chemical Shift δ (ppm) |
|---|---|---|
| 13 | 400MHz, CDCl₃ | 0.88(3H, t), 1.26(16H, brs), 1.63(2H, quin), 1.78(1H, m), 2.00(2H, m), 2.12(1H, m), 2.36(2H, t), 4.14(3H, m), 4.25(1H, dd), 4.38(1H, dd), 4.52(1H, dt), 4.72(1H, ddd), 4.76(1H, d) |
| 14 | 400MHz, CDCl₃ | 0.88(6H, t), 1.26(32H, brs), 1.61(4H, m), 1.79(2H, m), 2.04(2H, m), 2.31(4H, m), 4.14(2H, dt), 4.26(1H, dd), 4.39(1H, dd), 4.48(1H, ddd), 4.56(1H, ddd), 4.88(1H, d), 5.42(1H, ddd) |
| 15 | 400MHz, CDCl₃ | 0.88(3H, t), 1.25(24H, brs), 1.63(2H, quin), 1.77(1H, m), 2.00(2H, m), 2.13(1H, m), 2.35(2H, t), 4.14(3H, m), 4.25(1H, dd), 4.38(1H, dd), 4.52(1H, dt), 4.72(1H, ddd), 4.76(1H, d) |
| 16 | 400MHz, CDCl₃ | 0.88(6H, t), 1.25(48H, brs), 1.61(4H, m), 1.81(2H, m), 2.04(2H, m), 2.31(4H, t), 4.14(2H, m), 4.26(1H, dd), 4.39(1H, dd), 4.49(1H, m), 4.57(1H, m), 4.88(1H, d), 5.42(1H, m) |
| 17 | 400MHz, CDCl₃ | 0.84(3H, t), 1.25(25H, brs), 1.63(2H, m), 1.78(1H, m), 2.01(2H, m), 2.13(1H, m), 2.36(2H, t), 4.14(3H, m), 4.25(1H, dd), 4.38(1H, dd), 4.52(1H, dt), 4.72(1H, m), 4.76(1H, d) |
| 18 | 400MHz, CDCl₃ | 0.86(12H, t), 1.26(50H, brs), 1.59(4H, m), 1.80(2H, m), 2.03(2H, m), 2.31(4H, m), 4.14(2H, m), 4.26(1H, dd), 4.39(1H, dd), 4.48(1H, m), 4.57(1H, m), 4.89(1H, d), 5.42(1H, m) |
| 19 | 400MHz, CDCl₃ | 0.87(3H, t), 0.92(3H, t), 1.27(8H, brs), 1.59(4H, m), 1.79(2H, m), 2.02(2H, m), 2.28(4H, m), 4.13(2H, m), 4.25(1H, dd), 4.37(1H, dd), 4.48(1H, m), 4.56(1H, m), 4.87(1H, brs), 5.41(1H, dt) |
| 20 | 400MHz, CDCl₃ | 0.93(3H, t), 1.65(2H, m), 2.19(2H, quin), 2.32(2H, t), 4.09(1H, m), 4.20(3H, m), 4.32(3H, dd), 4.71(1H, d) |
| 21 | 400MHz, CDCl₃ | 0.93(6H, m), 1.62(4H, m), 2.14(2H, m), 2.27(2H, t), 2.29(2H, t), 3.99(1H, m), 4.13(1H, m), 4.22(2H, m), 4.36(2H, m), 4.82(1H, d), 5.39(1H, m) |

**[Table 5]**

| Synthesis Ex. No. | Measurement condition | Chemical Shift δ (ppm) |
|---|---|---|
| 22 | 400MHz, CDCl₃ | 0.88(3H, t), 1.29(8H, brs), 1.63(2H, quin), 2.22(2H, quin), 2.36(2H, t), 4.11(1H, m), 4.22(3H, m), 4.34(3H, m), 4.73(1H, d) |
| 23 | 400MHz, CDCl₃ | 0.88(6H, m), 1.28(16H, brs), 1.59(4H, quin), 2.15(2H, m), 2.29(2H, t), 2.31(2H, t), 4.00(1H, m), 4.13(1H, m), 4.28(2H, m), 4.37(2H, m), 4.83(1H, d), 5.40(1H, ddd) |
| 24 | 400MHz, CDCl₃ | 0.88(3H, t), 1.25(16H, brs), 1.63(2H, quin), 2.22(2H, quin), 2.36(2H, t), 4.11(1H, m), 4.22(3H, m), 4.34(3H, m), 4.73(1H, d) |
| 25 | 400MHz, CDCl₃ | 0.88(6H, m), 1.26(32H, brs), 1.59(4H, quin), 2.15(2H, m), 2.29(2H, t), 2.31(2H, t), 3.99(1H, m), 4.13(1H, m), 4.27(2H, m), 4.37(2H, m), 4.82(1H, d), 5.39(1H, ddd) |
| 26 | 400MHz, CDCl₃ | 0.88(3H, t), 1.25(24H, brs), 1.63(2H, quin), 2.21(2H, quin), 2.36(2H, t), 4.10(1H, m), 4.22(3H, m), 4.33(3H, m), 4.72(1H, d) |
| 27 | 400MHz, CDCl₃ | 0.88(6H, t), 1.25(48H, brs), 1.59(4H, quin), 2.15(2H, m), 2.29(2H, t), 2.31(2H, t), 4.00(1H, m), 4.13(1H, m), 4.27(2H, m), 4.37(2H, m), 4.82(1H, d), 5.40(1H, ddd) |
| 28 | 400MHz, CDCl₃ | 0.84(6H, t), 1.26(25H, brs), 1.63(2H, quin), 2.21(2H, quin), 2.36(2H, t), 4.11(1H, m), 4.22(3H, m), 4.33(3H, m), 4.73(1H, brs) |
| 29 | 400MHz, CDCl₃ | 0.85(12H, t), 1.25(50H, brs), 1.59(4H, quin), 2.15(2H, rn), 2.29(2H, t), 2.31(2H, t), 4.00(1H, m), 4.13(1H, m), 4.27(2H, m), 4.37(2H, m), 4.82(1H, brs), 5.39(1H, t) |
| 30 | 400MHz, CDCl₃ | 0.88(3H, t), 0.94(3H, t), 1.25(24H, brs), 1.61(4H, m), 2.16(2H, m), 2.29(2H, t), 2.31(2H, t), 4.01(1H, m), 4.14(1H, m), 4.28(2H, m), 4.38(2H, m), 4.83(1H, d), 5.40(1H, m) |
| 31 | 400MHz, CDCl₃ | 0.86(6H, t), 1.25(25H, brs), 1.63(2H, quin), 2.36(2H, t), 4.08(1H, m), 4.26(2H, m), 4.31(2H, dd), 4.39(2H, m), 4.91(1H, d) |
| 32 | 400MHz, CDCl₃ | 0.86(12H, t), 1.26(50H, brs), 1.60(4H, m), 2.32(4H, t), 4.23(4H, m), 4.39(2H, m), 5.02(1H, d), 5.36(1H, t) |

**[Table 6]**

| Synthesis Ex. No. | Measurement condition | Chemical Shift δ (ppm) |
|---|---|---|
| 33 | 400MHz, CD₃OD | 1.80(4H, m), 3.70(2H, m), 3.80(1H, dd), 3.83(1H, s), 3.96(1H, dt), 4.07(1H, dt), 4.33(1H, t), 4.38(1H, m), 4.53(1H, s) |
| 34 | 400MHz, CD₃OD | 1.71(1H, d), 2.19(1H, m), 3.66(1H, dd), 3.84(2H, m), 4.09(1H, m), 4.10(1H, s), 4.27(2H, m), 4.33(1H, m), 4.53(1H, s) |
| 35 | 400MHz, CD₃OD | 1.25(3H, s), 1.26(3H, s), 1.67-1.77(1H, m), 1.80-1.90(1H, mm), 1.94-2.02(2H, m), 3.91(1H, dd), 3.92-3.98(1H, m), 4.01-4.07(1H, m), 4.12(1H, dd), 4.28-4.32(1H, m), 4.44-4.50(1H, m), 4.54-4.60(1H, m), 4.91(1H, d) |
| 36 | 400MHz, CD₃OD | 1.26(6H, s), 2.10-2.14(2H, m), 3.88(1H, dd), 4.08-4.11(1H, m), 4.08-4.13(2H, m), 4.22-4.26(1H, m), 4.26-4.31(2H, m), 4.82(1H, d) |
| 37 | 400MHz, CD₃OD | 1.56-1.65(1H, m), 1.71-1.82(1H, m), 1.89-2.00(2H, m), 3.81-3.86(1H, m), 3.96-4.05(1H, m), 4.10-4.17(2H, m), 4.47-4.49(1H, m), 4.42-4.50(1H, m), 4.54-4.60(1H, m), 5.07(1H, d), 5.08(1H, d), 5.71 (1H, s), 5.77 (1H, s), 7.32-7.41(m, 5H) |
| 38 | 400MHz, CD₃OD | 2.07-2.12(2H, m), 3.93-4.08(2H, m), 4.11-4.13(2H, d), 4.23-4.39(2H, m), 4.41-4.46(1H, m), 4.99-5.00(1H, m), 5.70(1H, s), 5.74(1H, s), 7.35-7.42(5H, m) |
| 39 | 400MHz, CDCl₃ | 1.71-1.78(1H, m), 2.04-1.93(2H, m), 2.07-2.13(1H, m), 2.30-2.31(1H, m), 3.64-3.71(2H, m), 4.09-4.19(3H, m), 4.47-4.52(1H, m), 4.57(2H, s), 4.64-4.70(1H, m), 4.81 (1H, d), 7.26-7.38(5H, m) |

The products obtained in Synthesis Examples 1 to 39 were subjected to ^{1 3} C-NMR using a JNM-ECS400 (manufactured by JEOL Ltd.). The measurement results are shown in Tables 7 to 9.

**[Table 7]**

| Synthesis Ex. No. | Measurement condition | Chemical Shift δ (ppm) |
|---|---|---|
| 1 | 100MHz, CD₃OD | 63.2, 66.2, 68.8, 70.7, 76.9, 124.5, 156.1, 168.4 |
| 2 | 100MHz, CD₃OD | 33.7, 63.3, 70.4, 73.5, 75.7, 76.6, 123.7, 152.6, 171.5 |
| 3 | 100MHz, CD₃OD | 24.4, 30.4, 63.3, 70.4, 71.9, 75.5, 76.7, 120.4, 165.6, 173.2 |
| 4 | 100MHz, CD₃OD | 63.2, 70.3, 71.0, 75.8, 76.5, 77.4, 125.1, 154.3, 170.8 |
| 5 | 100MHz, CD₃OD | 61.1,63.1, 70.0, 70.6, 76.3, 76.8, 124.4, 155.8, 168.3 |
| 6 | 100MHz, CD₃OD | 42.4, 63.03, 70.3, 75.7, 75.8, 76.6, 77.8, 79.4, 124.0, 153.1, 170.7 |
| 7 | 100MHz, CDCl₃ | 22.4, 23.3, 60.9, 65.2, 72.0, 76.0, 78.7, 122.6, 147.3, 162.5 |
| 8 | 100MHz, CDCl₃ | 22.4, 22.7, 62.9, 70.5, 74.6, 74.8, 76.1, 122.6, 152.2, 166.7 |
| 9 | 100MHz, CDCl₃ | 13.60, 18.28, 22.87, 29.16, 35.88, 64.56, 67.87, 70.62, 74.48, 75.22, 119.61, 162.55, 170.10, 173.60 |
| 10 | 100MHz, CDCl₃ | 13.53, 13.59, 18.19, 18.35, 23.48, 28.99, 35.78, 61.86, 67.28, 70.76, 73.43, 74.44, 120.21, 160.49, 169.00, 172.11, 172.90 |
| 11 | 100MHz, CDCl₃ | 14.02, 22.54, 22.83, 24.77, 28.85, 29.00, 29.15, 31.58, 34.02, 64.54, 67.85, 70.61, 74.48, 75.23, 119.58, 162.60, 170.14, 173.78 |
| 12 | 100MHz, CDCl₃ | 14.04, 22.56, 23.51, 24.72, 24.87, 28.84, 28.87, 28.97, 29.00, 31.59, 33.94, 61.87, 67.28, 70.75, 73.43, 74.45, 120.22, 160.49, 169.02, 172.31, 173.10 |
| 13 | 100MHz, CDCl₃ | 14.08, 22.63, 22.87, 24.78, 29.07, 29.17, 29.20, 29.29, 29.40, 29.56, 31.85, 34.04, 64.58, 67.88, 70.61, 74.47, 75.19, 119.62, 162.53, 170.06, 173.80 |
| 14 | 100MHz, CDCl₃ | 14.08, 22.63, 23.50, 24.71, 24.87, 29.01, 29.05, 29.18, 29.21, 29.29, 29.40, 29.56, 33.92, 61.86, 67.27, 70.74, 73.42, 73.44, 120.21, 160.47, 168.98, 172.28, 173.07 |
| 15 | 100MHz, CDCl₃ | 14.09, 22.64, 22.87, 24.78, 29.08, 29.17, 29.32, 29.41, 29.57, 29.61, 29.64, 31.87, 34.04, 64.58, 67.88, 70.61, 74.47, 75.20, 119.62, 162.53, 170.06, 173.80 |

**[Table 8]**

| Synthesis Ex. No. | Measurement condition | Chemical Shift δ (ppm) |
|---|---|---|
| 16 | 100MHz, CDCl₃ | 14.09, 22.65, 23.51, 24.72, 24.88, 29.02, 29.06, 29.22, 29.33, 29.42, 29.62, 29.65, 31.88, 33.93, 61.88, 67.27, 70.74, 73.42, 74.44, 120.22, 160.48, 168.99, 172.29, 173.08 |
| 17 | 100MHz, CDCl₃ | 14.10, 19.66, 22.65, 22.87, 24.79, 27.04, 28.16, 29.09, 29.17, 29.22, 29.42, 29.57, 29.61, 29.65, 31.88, 32.69, 34.04, 37.04, 64.57, 67.87, 70.61, 74.47, 75.20, 119.61, 162.54, 170.09, 173.79 |
| 18 | 100MHz, CDCl₃ | 14.11, 19.67, 22.66, 23.52, 24.73, 24.89, 27.06, 29.02, 29.08, 29.24, 29.34, 29.44, 29.67, 30.00, 31.89, 32.71, 33.95, 37.06, 61.88, 67.28, 70.74, 73.43, 74.44, 120.23, 160.48, 168.99, 172.30, 173.09 |
| 19 | 100MHz, CDCl₃ | 13.55, 14.03, 18.37, 22.56, 23.50, 24.71, 28.86, 28.99, 31.59, 33.93, 35.80, 61.97, 67.28, 70.76, 73.43, 74.45, 120.22, 160.50, 169.01, 172.11, 173.10 |
| 20 | 100MHz, CDCl₃ | 13.59, 18.27, 32.49, 35.88, 64.63, 67.87, 72.34, 74.29, 75.19, 122.84, 149.64, 168.49, 173.63 |
| 21 | 100MHz, CDCl₃ | 13.58, 13.60, 18.22, 18.51, 32.56, 35.80, 62.11, 67.25, 72.50, 73.47, 74.22, 123.00, 148.20, 167.65, 172.29, 172.93 |
| 22 | 100MHz, CDCl₃ | 14.03, 22.54, 24.79, 28.86, 29.00, 31.59, 32.51, 34.05, 64.68, 67.93, 72.35, 74.30, 75.16, 122.87, 149.61, 168.43, 173.84 |
| 23 | 100MHz, CDCl₃ | 14.03, 22.55, 24.74, 25.04, 28.82, 28.87, 29.01, 31.59, 32.58, 33.95, 62.13, 67.26, 72.49, 73.47, 74.22, 123.03, 148.20, 167.63, 172.50, 173.12 |
| 24 | 100MHz, CDCl₃ | 14.09, 22.63, 24.79, 29.07, 29.20, 29.29, 29.40, 29.56, 31.85, 32.51, 34.05, 64.67, 67.92, 72.35, 74.29, 75.17, 122.87, 149.62, 168.45, 173.83 |
| 25 | 100MHz, CDCl₃ | 14.10, 22.65, 24.75, 25.06, 29.08, 29.18, 29.23, 29.31, 29.42, 29.57, 31.87, 33.96, 62.14, 67.26, 72.49, 73.47, 74.21, 123.04, 148.20, 167.63, 172.50, 173.13 |
| 26 | 100MHz, CDCl₃ | 14.11, 22.66, 24.81, 29.09, 29.23, 29.33, 29.43, 29.57, 29.62, 29.66, 31.89, 32.53, 34.06, 64.73, 68.00, 72.37, 74.30, 75.14, 122.90, 149.56, 168.35, 173.86 |
| 27 | 100MHz, CDCl₃ | 14.11, 22.66, 24.76, 25.06, 29.07, 29.09, 29.19, 29.24, 29.43, 29.59, 29.63, 29.67, 31.89, 32.59, 33.97, 62.14, 67.26, 72.49, 73.47, 74.21, 123.04, 148.20, 167.63, 172.51, 173.13 |

**[Table 9]**

| Synthesis Ex. No. | Measurement condition | Chemical Shift δ (ppm) |
|---|---|---|
| 28 | 100MHz, CDCl₃ | 14.09, 19.65, 22.64, 24.79, 27.03, 29.07, 29.21, 29.31, 29.41, 29.64, 29.98, 31.86, 32.50, 32.68, 34.04, 37.03, 64.66, 67.90, 72.34, 74.28, 75.17, 122.86, 149.62, 168.45, 173.81 |
| 29 | 100MHz, CDCl₃ | 14.10, 19.66, 22.65, 24.75, 25.06, 27.05, 29.08, 29.23, 29.33, 29.43, 29.66, 29.99, 31.88, 32.58, 32.70, 33.95, 37.05, 62.12, 67.26, 72.48, 73.46, 74.20, 123.03, 148.19,167.62, 172.49, 173.11 |
| 30 | 100MHz, CDCl₃ | 13.61, 14.09, 18.51, 22.63, 24.73, 29.04, 29.20, 29.32, 29.39, 29.57, 29.60, 29.64, 31.87, 32.56, 33.94, 35.81, 32.11, 67.25, 72.49, 73.46, 74.21, 123.01, 148.19, 167.64, 172.27, 173.12 |
| 31 | 100MHz, CDCl₃ | 14.13, 19.69, 22.68, 24.82, 27.07, 29.10, 29.24, 29.35, 29.44, 29.59, 29.64, 29.68, 29.94, 29.98, 30.01, 31.90, 32.71, 34.05, 37.07, 64.36, 64.91, 67.29, 68.57, 75.39, 123.76, 152.94, 165.13, 173.88 |
| 32 | 100MHz, CDCl₃ | 14.11, 19.67, 22.67, 24.76, 24.94, 26.71, 27.07, 29.07, 29.21, 29.25, 29.35, 29.45, 29.67, 30.00, 31.90, 32.71, 33.92, 33.95, 37.06, 61.76, 64.93, 67.19, 67.66, 73.44, 124.04, 151.76, 164.47, 172.38, 173.10 |
| 33 | 100MHz, CD₃OD | 28.13, 29.01, 64.69, 75.26, 75.61, 75.87, 79.10, 93.17, 109.47, 174.37 |
| 34 | 100MHz, CD₃OD | 29.58, 31.38, 69.58, 75.59, 76.74, 77.17, 82.02, 89.94, 107.83, 173.33 |
| 35 | 100MHz, CD₃OD | 22.6, 25.3, 25.8, 28.6, 64.9, 70.5, 73.3, 74.1, 74.1, 109.3, 119.0, 162.5, 169.1 |
| 36 | 100MHz, CD₃OD | 25.4, 25.8, 32.2, 65.0, 71.9, 73.5, 74.1, 74.2, 109.3, 122.2, 150.5, 167.7 |
| 37 | 100MHz, CD₃OD | 22.5, 22.7, 28.6, 28.6, 66.2, 66.3, 70.5, 70.6, 73.3, 74.0, 74.1, 74.3, 103.7, 104.3, 119.0, 119.1, 126.6, 127.0, 128.2, 128.4, 129.4, 129.6, 136.8, 137.5, 162.4, 168.9, 169.1 |
| 38 | 100MHz, CD₃OD | 32.3, 66.3, 66.6, 72.1, 73.5, 74.0, 74.3, 74.4, 103.8, 104.4, 122.2, 122.3, 126.9, 127.3, 128.4, 128.5, 129.6, 129.8, 137.0, 137.5, 150.4, 150.6, 167.7, 167.9 |
| 39 | 100MHz, CD₃OD | 22.9, 29.2, 68.2, 70.1, 70.6, 73.5, 74.4, 74.8, 119.4, 127.8, 127.9, 128.5, 137.5, 162.9, 170.1 |

### Test Example 1 [Stability test -1]

For each of the samples of Synthesis Examples 1 to 6, 1% aqueous solutions were adjusted to pH 3, 5, 7, 8, and 9 with a dilute potassium hydroxide aqueous solution, and then placed in 50 mL screw tubes and
2: Color changes compared to immediately after preparation.
1: Strongly colored compared to immediately after preparation. Based on the results of the above evaluation, the following classification was made:
   ○: Total score of 10 panelists is 25 or more
   △: Total score of 10 panelists is 16-24
   ×: The total score of 10 panelists is 15 or less

**[Table 10]**

| Example No. | Ascorbic acid derivatives | Residual Rate | After 2 weeks | After 4 weeks |
|---|---|---|---|---|
| Comparison 1 | Ascorbic acid | pH3 | ○ | Δ |
| | | pH5 | ○ | × |
| | | pH7 | × | × |
| | | pH8 | × | × |
| | | pH9 | × | × |
| Comparison 2 | Bisglyceryl ascorbic acid | pH3 | ⊚ | ⊚ |
| | | pH5 | ⊚ | ⊚ |
| | | pH7 | × | × |
| | | pH8 | × | × |
| | | pH9 | × | × |
| 1 | 2,3-O-(1,2-ethanediyl) ascorbic acid (synthesis example 1) | pH3 | ⊚ | ⊚ |
| | | pH5 | ⊚ | ⊚ |
| | | pH7 | ○ | Δ |
| | | pH8 | Δ | × |
| | | pH9 | × | × |
| 2 | 2,3-O-(1,3-propanediyl) ascorbic acid (synthesis example 2) | pH3 | ⊚ | ⊚ |
| | | pH5 | ⊚ | ⊚ |
| | | pH7 | ⊚ | ⊚ |
| | | pH8 | ⊚ | ○ |
| | | pH9 | ○ | Δ |
| 3 | 2,3-O-(1,4-butanediyl) ascorbic acid (synthesis example 3) | pH3 | ⊚ | ⊚ |
| | | pH5 | ⊚ | ⊚ |
| | | pH7 | ⊚ | ⊚ |
| | | pH8 | ⊚ | ○ |
| | | pH9 | ○ | Δ |
| 4 | 2,3-O-(2-hydroxypropane-1,3-diyl) ascorbic acid (Synthesis example 4) | pH3 | ⊚ | ⊚ |
| | | pH5 | ⊚ | ⊚ |
| | | pH7 | ○ | ○ |
| | | pH8 | ○ | Δ |
| | | pH9 | ○ | Δ |
| 5 | 2,3-O-(1-hydroxymethyl-1,2-ethanediyl) ascorbic acid (synthesis example 5) | pH3 | ⊚ | ⊚ |
| | | pH5 | ⊚ | ⊚ |
| | | pH7 | ○ | Δ |
| | | pH8 | ○ | Δ |
| | | pH9 | ○ | Δ |
| 6 | 2,3-O-(3,3-dimethyleneoxetane) ascorbic acid (synthesis example 6) | pH3 | ⊚ | ⊚ |
| | | pH5 | ⊚ | ⊚ |
| | | pH7 | ○ | ○ |
| | | pH8 | ○ | Δ |
| | | pH9 | ○ | Δ |

**[Table 11]**

| Example No. | Ascorbic acid derivatives | | After 2 weeks | After 4 weeks |
|---|---|---|---|---|
| Comparison 3 | Ascorbic acid | Odor | Δ | × |
| | | Coloration | Δ | × |
| Comparison 4 | Bisglyceryl ascorbic acid | Odor | Δ | Δ |
| | | Coloration | Δ | Δ |
| 7 | 2,3-O-(1,2-ethanediyl) ascorbic acid (synthesis example 1) | Odor | ○ | ○ |
| | | Coloration | ○ | Δ |
| 8 | 2,3-O-(1,3-propanediyl) ascorbic acid (synthesis example 2) | Odor | ○ | Δ |
| | | Coloration | ○ | ○ |
| 9 | 2,3-O-(1,4-butanediyl) ascorbic acid (synthesis example 3) | Odor | ○ | ○ |
| | | Coloration | ○ | ○ |
| 10 | 2,3-O-(2-hydroxypropane-1,3-diyl) ascorbic acid (synthesis example 4) | Odor | ○ | ○ |
| | | Coloration | ○ | ○ |
| 11 | 2,3-O-(1-hydroxymethyl-1,2-ethanediyl) ascorbic acid (synthesis example 5) | Odor | ○ | ○ |
| | | Coloration | ○ | ○ |
| 12 | 2,3-O-(3,3-dimethyleneoxetane) ascorbic acid (synthesis example 6) | Odor | ○ | ○ |
| | | Coloration | ○ | ○ |

### Test Example 2 [Stability test -2]

For each of the samples of Synthesis Examples 9 to 32, (a) and (b) shown in Table 12 were each heated and mixed. After cooling, (c) shown in Table 12 was added to prepare a cream with a pH of 7 or 9. The sample was stored at 50°C for 4 weeks, and the residual rate was determined in the same manner as in Test Example 1 and evaluated according to the criteria described below. The odor and coloration were also evaluated according to the same method and criteria as in Test Example 1. The results regarding the residual rate are shown in Tables 13 and 14, and the results regarding the odor and coloration are shown in Tables 15 and 16.

### Residual rates:

⊚: 95% or more
○: 85% or more, less than 95%
×: Less than 85%

**[Table 12]**

| Ingredients | | Blend ratio (W/W%) |
|---|---|---|
| (a) | Cetearyl alcohol | 4.0 |
| | Glyceryl Stearate | 2.5 |
| | PEG-40 Stearate | 1.5 |
| | Squalene | 10.0 |
| (b) | Butylene Glycol (BG) | 5.0 |
| | Phosphate buffer (pH 7.0) or Tris buffer (pH 9.0) | 1.0 |
| | Phenoxyethanol | 0.3 |
| | Water | 70.7 |
| (c) | Butylene Glycol (BG) | 1.0 |
| | Ascorbic acid derivative of the present invention | 4.0 |

**[Table 13]**

| Example No. | Ascorbic acid derivatives | | Residual Rate | |
|---|---|---|---|---|
| | | | After 2 weeks | After 4 weeks |
| Comparison 5 | Ascorbyl tetrahexyldecanoate | pH7 | ○ | × |
| | | pH9 | ○ | × |
| 13 | 6-O-butanoyl-2,3-O-(1,4-butanediyl) ascorbic acid (synthesis example 9) | pH7 | ⊚ | ⊚ |
| | | pH9 | ⊚ | ⊚ |
| 14 | 5,6-O-dibutanoyl-2,3-O-(1,4-butanediyl) ascorbic acid (synthesis example 10) | pH7 | ⊚ | ⊚ |
| | | pH9 | ⊚ | ⊚ |
| 15 | 6-O-octanoyl-2,3-O-(1,4-butanediyl) ascorbic acid (synthesis example 11) | pH7 | ⊚ | ⊚ |
| | | pH9 | ⊚ | ○ |
| 16 | 5,6-O-dioctanoyl-2,3-O-(1,4-butanediyl) ascorbic acid (synthetic example 12) | pH7 | ⊚ | ⊚ |
| | | pH9 | ⊚ | ⊚ |
| 17 | 6-O-lauroyl-2,3-O-(1,4-butanediyl) ascorbic acid (synthesis example 13) | pH7 | ⊚ | ⊚ |
| | | pH9 | ⊚ | ⊚ |
| 18 | 5,6-O-dilauroyl-2,3-O-(1,4-butanediyl) ascorbic acid (synthesis example 14) | pH7 | ⊚ | ⊚ |
| | | pH9 | ⊚ | ○ |
| 19 | 6-O-palmitoyl-2,3-O-(1,4-butanediyl) ascorbic acid (synthetic example 15) | pH7 | ⊚ | ⊚ |
| | | pH9 | ⊚ | ⊚ |
| 20 | 5,6-O-dipalmitoyl-2,3-O-(1,4-butanediyl) ascorbic acid (synthetic example 16) | pH7 | ⊚ | ⊚ |
| | | pH9 | ⊚ | ⊚ |
| 21 | 6-O-isostearoyl-2,3-O-(1,4-butanediyl) ascorbic acid (synthesis example 17) | pH7 | ⊚ | ⊚ |
| | | pH9 | ⊚ | ⊚ |
| 22 | 5,6-O-diisostearoyl-2,3-O-(1,4-butanediyl) ascorbic acid (synthesis example 18) | pH7 | ⊚ | ⊚ |
| | | pH9 | ⊚ | ⊚ |

**[Table 14]**

| Example No. | Ascorbic acid derivatives | | Residual Rate | |
|---|---|---|---|---|
| | | | After 2 weeks | After 4 weeks |
| 23 | 5-O-butanoyl-6-O-octanoyl-2,3-(1,4-butanediyl) ascorbic acid (synthetic example 19) | pH7 | ⊚ | ⊚ |
| | | pH9 | ⊚ | ⊚ |
| 24 | 6-O-butanoyl-2,3-O-(1,3-propanediyl) ascorbic acid (synthesis example 20) | pH7 | ⊚ | ⊚ |
| | | pH9 | ⊚ | ⊚ |
| 25 | 5,6-O-dibutanoyl-2,3-O-(1,3-propanediyl) ascorbic acid (synthesis example 21) | pH7 | ⊚ | ⊚ |
| | | pH9 | ⊚ | ⊚ |
| 26 | 6-O-octanoyl-2,3-O-(1,3-propanediyl) ascorbic acid (synthesis example 22) | pH7 | ⊚ | ⊚ |
| | | pH9 | ⊚ | ⊚ |
| 27 | 5,6-O-dioctanoyl-2,3-O-(1,3-propanediyl) ascorbic acid (synthesis example 23) | pH7 | ⊚ | ⊚ |
| | | pH9 | ⊚ | ⊚ |
| 28 | 6-O-lauroyl-2,3-O-(1,3-propanediy) ascorbic acid (synthesis example 24) | pH7 | ⊚ | ⊚ |
| | | pH9 | ⊚ | ⊚ |
| 29 | 5,6-O-dilauroyl-2,3-O-(1,3-propanediyl) ascorbic acid (Synthesis example 25) | pH7 | ⊚ | ⊚ |
| | | pH9 | ⊚ | ⊚ |
| 30 | 6-O-palmitoyl-2,3-O-(1,3-propanediyl) ascorbic acid (synthesis example 26) | pH7 | ⊚ | ⊚ |
| | | pH9 | ⊚ | ○ |
| 31 | 5,6-O-dipalmitoyl-2,3-O-(1,3-propanediyl) ascorbic acid (Synthesis Example 27) | pH7 | ⊚ | ⊚ |
| | | pH9 | ⊚ | ⊚ |
| 32 | 6-O-isostearoyl-2,3-O-(1,3-propanediyl) ascorbic acid (synthetic example 28) | pH7 | ⊚ | ⊚ |
| | | pH9 | ⊚ | ⊚ |
| 33 | 5,6-O-diisostearoyl-2,3-O-(1,3-propanediyl) ascorbic acid (synthesis example 29) | pH7 | ⊚ | ⊚ |
| | | pH9 | ⊚ | ⊚ |
| 34 | 5-butanoyl-6-O-palmitoyl-2,3-O-(1,3-propanediyl) ascorbic acid (Synthesis Example 30) | pH7 | ⊚ | ⊚ |
| | | pH9 | ⊚ | ⊚ |
| 35 | 6-O-isostearoyl-2,3-O-(1,2-ethanediyl) ascorbic acid (synthesis example 31) | pH7 | ⊚ | ⊚ |
| | | pH9 | ⊚ | ⊚ |
| 36 | 5,6-O-diisostearoyl-2,3-O-(1,2-ethanediyl) ascorbic acid (Synthesis Example 32) | pH7 | ⊚ | ⊚ |
| | | pH9 | ⊚ | ⊚ |

**[Table 15]**

| Example No. | Ascorbic acid derivatives | | After 2 weeks | After 4 weeks |
|---|---|---|---|---|
| Comparison 6 | Ascorbyl tetrahexyldecanoate | Odor | ○ | Δ |
| | | Coloration | ○ | Δ |
| 37 | 6-O-butanoyl-2,3-O-(1,4-butanediyl) ascorbic acid (synthesis example 9) | Odor | ○ | Δ |
| | | Coloration | ○ | ○ |
| 38 | 5,6-O-dibutanoyl-2,3-O-(1,4-butanediyl) ascorbic acid (synthesis example 10) | Odor | ○ | Δ |
| | | Coloration | ○ | ○ |
| 39 | 6-O-octanoyl-2,3-O-(1,4-butanediyl) ascorbic acid (synthesis example 11) | Odor | ○ | Δ |
| | | Coloration | ○ | ○ |
| 40 | 5,6-O-dioctanoyl-2,3-O-(1,4-butanediyl) ascorbic acid (synthetic example 12) | Odor | ○ | Δ |
| | | Coloration | ○ | ○ |
| 41 | 6-O-lauroyl-2,3-O-(1,4-butanediyl) ascorbic acid (synthesis example 13) | Odor | ○ | Δ |
| | | Coloration | ○ | ○ |
| 42 | 5,6-O-dilauroyl-2,3-O-(1,4-butanediyl) ascorbic acid (synthesis example 14) | Odor | ○ | Δ |
| | | Coloration | ○ | ○ |
| 43 | 6-O-palmitoyl-2,3-O-(1,4-butanediyl) ascorbic acid (synthetic example 15) | Odor | ○ | Δ |
| | | Coloration | ○ | ○ |
| 44 | 5,6-O-dipalmitoyl-2,3-O-(1,4-butanediyl) ascorbic acid (synthetic example 16) | Odor | ○ | Δ |
| | | Coloration | ○ | ○ |
| 45 | 6-O-isostearoyl-2,3-O-(1,4-butanediyl) ascorbic acid (synthesis example 17) | Odor | ○ | Δ |
| | | Coloration | ○ | ○ |
| 46 | 5,6-O-diisostearoyl-2,3-O-(1,4-butanediyl) ascorbic acid (synthesis example 18) | Odor | ○ | Δ |
| | | Coloration | ○ | ○ |
| 47 | 5-O-butanoyl-6-O-octanoyl-2,3-(1,4-butanediyl) ascorbic acid (synthetic example 19) | Odor | ○ | Δ |
| | | Coloration | ○ | ○ |
| 48 | 6-O-butanoyl-2,3-O-(1,3-propanediyl) ascorbic acid (synthesis example 20) | Odor | ○ | Δ |
| | | Coloration | ○ | ○ |
| 49 | 5,6-O-dibutanoyl-2,3-O-(1,3-propanediyl) ascorbic acid (synthesis example 21) | Odor | ○ | Δ |
| | | Coloration | ○ | ○ |
| 50 | 6-O-octanoyl-2,3-O-(1,3-propanediyl) ascorbic acid (synthesis example 22) | Odor | ○ | Δ |
| | | Coloration | ○ | ○ |

**[Table 16]**

| Example No. | Ascorbic acid derivatives | | After 2 weeks | After 4 weeks |
|---|---|---|---|---|
| 51 | 5,6-O-dioctanoyl-2,3-O-(1,3-propanediyl) ascorbic acid (synthesis example 23) | Odor | ○ | Δ |
| | | Coloration | ○ | ○ |
| 52 | 6-O-lauroyl-2,3-O-(1,3-propanediyl) ascorbic acid (synthesis example 24) | Odor | ○ | Δ |
| | | Coloration | ○ | ○ |
| 53 | 5,6-O-dilauroyl-2,3-O-(1,3-propanediyl) ascorbic acid (Synthesis example 25) | Odor | ○ | Δ |
| | | Coloration | ○ | Δ |
| 54 | 6-O-palmitoyl-2,3-O-(1,3-propanediyl) ascorbic acid (synthesis example 26) | Odor | ○ | Δ |
| | | Coloration | ○ | Δ |
| 55 | 5,6-O-dipalmitoyl-2,3-O-(1,3-propanediyl) ascorbic acid (Synthesis Example 27) | Odor | ○ | Δ |
| | | Coloration | ○ | Δ |
| 56 | 6-O-isostearoyl-2,3-O-(1,3-propanediyl) ascorbic acid (synthetic example 28) | Odor | ○ | Δ |
| | | Coloration | ○ | ○ |
| 57 | 5,6-O-diisostearoyl-2,3-O-(1,3-propanediyl) ascorbic acid (synthesis example 29) | Odor | ○ | Δ |
| | | Coloration | ○ | ○ |
| 58 | 5-butarloyl-6-O-palmitoyl-2,3-O-(1,3-propanediyl) ascorbic acid (Synthesis Example 30) | Odor | ○ | Δ |
| | | Coloration | ○ | ○ |
| 59 | 6-O-isostearoyl-2,3-O-(1,2-ethanediyl) ascorbic acid (synthesis example 31) | Odor | ○ | Δ |
| | | Coloration | ○ | ○ |
| 60 | 5,6-O-diisostearoyl-2,3-O-(1,2-ethanediyl) ascorbic acid (Synthesis Example 32) | Odor | ○ | Δ |
| | | Coloration | ○ | ○ |

The results of the above test examples show that the ascorbic acid derivatives represented by the general formula (1) of the present invention exhibit excellent stability over time, with no decrease in residual rate compared to ascorbic acid and conventional ascorbic acid derivatives when stored at 50°C, and are also substantially free of odor and coloration. In Test Example 1, in the neutral to weakly alkaline range of pH 7 to 9, the residual rates of ascorbic acid and conventional ascorbic acid derivatives decreased to less than 30% after two weeks, whereas the ascorbic acid derivatives represented by the general formula (1) of the present invention maintained a residual rate of 50% or more after two weeks, and even after four weeks, the residual rates were far higher than those of ascorbic acid and conventional ascorbic acid derivatives.

Similarly, in Test Example 2, it was clear that the ascorbic acid derivative represented by general formula (1) of the present invention has higher stability than ascorbyl tetrahexyldecanoate, a conventional ascorbic acid derivative, in the neutral to weakly alkaline range of pH 7 to 9. The ascorbic acid derivative represented by general formula (1) of the present invention has the excellent properties inherent to ascorbic acid, but the results shown in Tables 10, 11, and 13 to 16 further demonstrate that the stability over time, which was a problem with conventional ascorbic acid derivatives, has been improved, making it more suitable as a component material for cosmetics.

### Test Example 3 [Collagen production promoting effect]

Normal human dermal fibroblasts were prepared in D-MEM containing 5% (v/v) fetal bovine serum to a cell density of 2.5 × 10⁴ cell s /well, and then pre-incubated for 24 hours on a 96-well plate. After removing the medium, samples prepared to a predetermined concentration in D-MEM containing 5% (v/v) fetal bovine serum were added to each well and cultured for 48 hours. After the culture was completed, the amount of free collagen in the supernatant was quantified by ELISA. Measurements were performed in triplicate (N=3).

The amount of collagen produced when the sample was measured at a concentration of 10 mM or less was compared with that of the control group, and the results (% values when the control group was set to 100%) are shown in Table 17 based on the following criteria.
<100%: ±
100-140%: +
140%<: ++

**[Table 17]**

| Example No. | Ascorbic acid derivatives | Collagen production-promoting effect |
|---|---|---|
| Comparison 7 | 2-O-glyceryl ascorbic acid | + |
| Comparison 8 | 3-O-glyceryl ascorbic acid | + |
| 61 | 2,3-O-(1,2-ethanediyl) ascorbic acid (synthesis example 1) | + |
| 62 | 2,3-O-(1,3-propanediyl) ascorbic acid (synthesis example 2) | ++ |
| 63 | 2,3-O-(1,4-butanediyl) ascorbic acid (synthesis example 3) | + |
| 64 | 2,3-O-(2-hydroxypropane-1,3-diyl) ascorbic acid (synthesis example 4) | + |
| 65 | 2,3-O-(1-hydroxymethyl-1,2-ethanediyl) ascorbic acid (synthesis example 5) | + |
| 66 | 2,3-O-(3,3-dimethyleneoxetane) ascorbic acid (synthesis example 6) | ++ |
| 67 | 6-O-butanoyl-2,3-O-(1,4-butanediyl) ascorbic acid (synthesis example 9) | + |
| 68 | 5,6-O-dibutanoyl-2,3-O-(1,4-butanediyl) ascorbic acid (synthesis example 10) | ++ |
| 69 | 5,6-O-dioctanoyl-2,3-O-(1,4-butanediyl) ascorbic acid (synthetic example 12) | ++ |
| 70 | 6-O-palmitoyl-2,3-O-(1,4-butanediyl) ascorbic acid (synthetic example 15) | ++ |
| 71 | 6-O-isostearoyl-2,3-O-(1,4-butanediyl) ascorbic acid (synthesis example 17) | ++ |
| 72 | 5,6-O-dibutanoyl-2,3-O-(1,3-propanediyl) ascorbic acid (synthesis example 21) | ++ |
| 73 | 5,6-O-dioctanoyl-2,3-O-(1,3-propanediyl) ascorbic acid (synthesis example 23) | ++ |
| 74 | 5,6-O-dipalmitoyl-2,3-O-(1,3-propanediyl) ascorbic acid (synthesis Example 27) | ++ |
| 75 | 5,6-O-diisostearoyl-2,3-O-(1,3-propanediyl) ascorbic acid (synthesis example 29) | ++ |

The results in Table 17 clearly show that the ascorbic acid derivative represented by the general formula (1) of the present invention has a collagen production-promoting effect equal to or greater than that of known ascorbic acid derivatives, i.e., 2-O-glyceryl ascorbic acid and 3-O-glyceryl ascorbic acid.

### Test Example 4 [Hyaluronic acid production promoting effect]

Normal human dermal fibroblasts (NHDF) were prepared in D-MEM containing 5% (v/v) fetal bovine serum to a cell density of 2.5 × 10⁴ cells/well, and then pre-incubated on a 96-well plate for 24 hours. After removing the medium, samples prepared in serum-free D-MEM were added to each well and cultured for 48 hours. After the culture was completed, the amount of hyaluronic acid in the supernatant was quantified by ELISA. Measurements were performed in triplicate (N=3).

The amount of hyaluronic acid produced when the sample was measured at a concentration of 10 mM or less was compared with that of the control group, and the results (% values when the control group is set to 100%) are shown in Tables 18 to 20 based on the following criteria.
<100%: ±
100-120%: +
120%<: ++

**[Table 18]**

| Example No. | Ascorbic acid derivatives | Hyaluronic acid production-promoting effect |
|---|---|---|
| Comparison 9 | Ascorbic acid | ± |
| Comparison 10 | Ascorbyl phosphate 3Na palmitate | ± |
| 76 | 2,3-O-(1,2-ethanediyl) ascorbic acid (synthesis example 1) | + |
| 77 | 2,3-O-(1,3-propanediyl) ascorbic acid (synthesis example 2) | ++ |
| 78 | 2,3-O-(1,4-butanediyl) ascorbic acid (synthesis example 3) | + |
| 79 | 2,3-O-(2-hydroxypropane-1,3-diyl) ascorbic acid (synthesis example 4) | ++ |
| 80 | 2,3-O-(1-hydroxymethyl-1,2-ethanediyl) ascorbic acid (synthesis example 5) | + |
| 81 | 2,3-O-(3,3-dimethyleneoxetane) ascorbic acid (synthesis example 6) | ++ |
| 82 | 2,3-O-(2,2-dimethyl-1,2-ethanediyl) ascorbic acid (synthesis example 7) | + |
| 83 | 2,3-O-(1,1-dimethyl-1,2-ethanediyl) ascorbic acid (synthetic example 8) | + |
| 84 | 6-O-butanoyl-2,3-O-(1,4-butanediyl) ascorbic acid (synthesis example 9) | ++ |
| 85 | 5,6-O-dibutanoyl-2,3-O-(1,4-butanediyl) ascorbic acid (synthesis example 10) | ++ |
| 86 | 6-O-octanoyl-2,3-O-(1,4-butanediyl) ascorbic acid (synthesis example 11) | ++ |
| 87 | 5,6-O-dioctanoyl-2,3-O-(1,4-butanediyl) ascorbic acid (synthetic example 12) | ++ |
| 88 | 6-O-lauroyl-2,3-O-(1,4-butanediyl) ascorbic acid (synthesis example 13) | ++ |
| 89 | 5,6-O-dilauroyl-2,3-O-(1,4-butanediyl) ascorbic acid (synthesis example 14) | ++ |
| 90 | 6-O-palmitoyl-2,3-O-(1,4-butanediyl) ascorbic acid (synthetic example 15) | ++ |

**[Table 19]**

| Example No. | Ascorbic acid derivatives | Hyaluronic acid production-promoting effect |
|---|---|---|
| 91 | 5,6-O-dipalmitoyl-2,3-O-(1,4-butanediyl) ascorbic acid (synthetic example 16) | ++ |
| 92 | 6-O-isostearoyl-2,3-O-(1,4-butanediyl) ascorbic acid (synthesis example 17) | ++ |
| 93 | 5,6-O-diisostearoyl-2,3-O-(1,4-butanediyl) ascorbic acid (synthesis example 18) | ++ |
| 94 | 6-O-butanoyl-2,3-O-(1,3-propanediyl) ascorbic acid (synthesis example 20) | ++ |
| 95 | 5,6-O-dibutanoyl-2,3-O-(1,3-propanediyl) ascorbic acid (synthesis example 21) | ++ |
| 96 | 6-O-octanoyl-2,3-O-(1,3-propanediyl) ascorbic acid (synthesis example 22) | ++ |
| 97 | 5,6-O-dioctanoyl-2,3-O-(1,3-propanediyl) ascorbic acid (synthesis example 23) | + |
| 98 | 6-O-lauroyl-2,3-O-(1,3-propanediyl) ascorbic acid (synthesis example 24) | ++ |
| 99 | 5,6-O-dilauroyl-2,3-O-(1,3-propanediyl) ascorbic acid (synthesis example 25) | ++ |
| 100 | 6-O-palmitoyl-2,3-O-(1,3-propanediyl) ascorbic acid (synthesis example 26) | ++ |
| 101 | 5,6-O-dipalmitoyl-2,3-O-(1,3-propanediyl) ascorbic acid (synthesis Example 27) | ++ |
| 102 | 6-O-isostearoyl-2,3-O-(1,3-propanediyl) ascorbic acid (synthetic example 28) | ++ |
| 103 | 5,6-O-diisostearoyl-2,3-O-(1,3-propanediyl) ascorbic acid (synthesis example 29) | + |
| 104 | 6-O-isostearoyl-2,3-O-(1,2-ethanediyl) ascorbic acid (synthesis example 31) | ++ |
| 105 | 5,6-O-diisostearoyl-2,3-O-(1,2-ethanediyl) ascorbic acid (synthesis Example 32) | + |

**[Table 20]**

| Example No. | Ascorbic acid derivatives | Hyaluronic acid production-promoting effect |
|---|---|---|
| 106 | 4-hydroxy-2,6,9,14-tetraoxatricyclo [6.6.0.0^{1.5}] tetradecane-7-one (synthesis Example 33) | + |
| 107 | 4-hydroxy-2,6,9,13-tetraoxatricydo [6.5.0.0^{1.5}] tridecane-7-one (synthesis Example 34) | + |
| 108 | 5,6-O-isopropylidene-2,3-O-(1,4-butanediyl) ascorbic acid (synthesis example 35) | ++ |
| 109 | 5,6-O-isopropylidene-2,3-O-(1,3-propanediyl) ascorbic acid (synthesis example 36) | ++ |
| 110 | 5,6-O-(phenylmethylene)-2,3-O-(1,4-butanediyl) ascorbic acid (synthesis example 37) | + |
| 111 | 5,6-O-(phenylmethylene)-2,3-O-(1,3-propane-diyl) ascorbic acid (synthesis example 38) | ++ |
| 112 | 6-O-benzyl-2,3-O-(1,4-butanediyl) ascorbic acid (synthetic example 39) | + |

The results in Tables 18 to 20 clearly show that the ascorbic acid derivatives of the present invention have a higher hyaluronic acid production promoting effect than ascorbic acid and known ascorbic acid derivatives.

### Test Example 5 [Antioxidant effect]

Normal human epidermal keratinocytes were seeded onto a 96-well plate using KG2 medium at a cell density of 2.0 × 10⁴ cells/well. After 24 hours of pre-incubation, samples adjusted to a predetermined concentration in KG2 medium were added to each well. After 24 hours of incubation, the medium was removed, followed by washing with HBSS(-) and the incorporation of the ROS-reactive fluorescent probe DCFHDA for 30 minutes. The cells were again washed with HBSS(-), treated with 0.2 mM H₂O₂, and incubated for 2 hours. The fluorescence intensity was measured, and the amount of ROS produced per unit protein was calculated by dividing the fluorescence intensity by the amount of protein quantified by the BCA method, resulting in an analytical value of 1. Furthermore, the amount of ROS production calculated without adding the sample adjusted to a predetermined concentration using the above test method was set to analytical value 2; and the amount of ROS production calculated without adding the sample or 0.2 mM H₂O₂ was set to analytical value 3.

ROS production inhibition rate (%) was calculated using the following formula. The results are shown in Table 21. The higher the ROS production inhibition rate, the greater the antioxidant effect can be evaluated to be.

ROS production inhibition rate (%) = [(analysis value 2) - (analysis value 1)] / [(analysis value 2) - (analysis value 3)] × 100

The ROS production inhibition rate when the sample was measured at a concentration of 10 mM or less was evaluated as follows. Note that the measurement was performed in N=4.
<20%: +
20-40%: ++
40%<: +++

**[Table 21]**

| Example No. | Ascorbic acid derivatives | Antioxidant effect |
|---|---|---|
| Comparison 11 | 2-O-glyceryl ascorbic acid | + |
| Comparison 12 | 3-O-glyceryl ascorbic acid | + |
| 113 | 6-O-butanoyl-2,3-O-(1,4-butanediyl) ascorbic acid (synthesis example 9) | ++ |
| 114 | 5,6-O-dibutanoyl-2,3-O-(1,4-butanediyl) ascorbic acid (synthesis example 10) | + |
| 115 | 6-O-lauroyl-2,3-O-(1,4-butanediyl) ascorbic acid (synthesis example 13) | + |
| 116 | 5,6-O-dilauroyl-2,3-O-(1,4-butanediyl) ascorbic acid (synthesis example 14) | + |
| 117 | 6-O-palmitoyl-2,3-O-(1,4-butanediyl) ascorbic acid (synthetic example 15) | + |
| 118 | 6-O-isostearoyl-2,3-O-(1,4-butanediyl) ascorbic acid (synthesis example 17) | + |
| 119 | 5,6-O-dioctanoyl-2,3-O-(1,3-propanediyl) ascorbic acid (synthesis example 23) | + |
| 120 | 4-hydroxy-2,6,9,14-tetraoxatricyclo [6.6.0.0^{1.5}] tetradecane-7-one (synthesis Example 33) | ++ |
| 121 | 4-hydroxy-2,6,9,13-tetraoxatricyclo [6.5.0.0^{1.5}] tridecane-7-one (synthesis Example 34) | ++ |

The results in Table 21 show that the ascorbic acid derivatives of the present invention have a high antioxidant effect equal to or greater than that of known ascorbic acid derivatives.

### Example 122 Cream

Raw materials for an oil phase, ingredients (1) to (5) and raw materials for an aqueous phase, ingredients (6) to (10), shown in Table 22 were each heated to 70°C and dissolved, and the oil phase and the aqueous phase were prepared, respectively. The oil phase was then added to the aqueous phase and pre-emulsified, and then the mixture was homogeneously emulsified using a homo-mixer. Thereafter, the mixture was cooled to room temperature while stirring thoroughly to prepare a cream. In Table 22 and subsequent tables, the blend amounts are in parts by mass.

**[Table 22]**

| | No | Ingredient name | Blend Amount |
|---|---|---|---|
| Oil phase | 1 | Squalene | 9.0 |
| | 2 | Vaseline | 6.0 |
| | 3 | Stearyl alcohol | 5.0 |
| | 4 | Polyoxyethylene(25) cetyl ether | 2.5 |
| | 5 | Glyceryl monostearate | 1.5 |
| Aqueous phase | 6 | 2,3-O-(1,2-ethanediyl) ascorbic acid (synthesis example 1) | 2.0 |
| | 7 | Glycerin | 6.0 |
| | 8 | Preservative | Appropriate amount |
| | 9 | pH Adjuster | Appropriate amount |
| | 10 | Purified water | Remainder* |

| | | | |
|---|---|---|---|
| * Refers to the amount required to make the total blend amount to 100 masses. The same is true in the table below. | | | |

### Example 123 Milky lotion

The ingredients (1) to (9) for oil phase and the ingredients (10) to (13) for aqueous phase in the compositions shown in Table 23 were each heated to 70°C and dissolved, and the oil phase and the aqueous phase were prepared, respectively. The oil phase was then added to the aqueous phase and pre-emulsified, and the mixture was homogeneously emulsified using a homo-mixer. The mixture was then cooled to room temperature while stirring well to prepare a milky lotion.

**[Table 23]**

| | No | Ingredient name | Blend Amount |
|---|---|---|---|
| | 1 | Isostearyl palmitate | 5.0 |
| | 2 | Jojoba oil | 2.0 |
| | 3 | Dimethyl polysiloxane | 2.0 |
| | 4 | Cetanol | 1.0 |
| Oil phase | 5 | Stearic acid | 1.5 |
| | 6 | Beeswax | 2.5 |
| | 7 | Paraffin Wax | 2.5 |
| | 8 | Polyoxyethylene monostearate (20) sorbitan | 1.2 |
| | 9 | Polyoxyethylene tetraoleiate (40) sorbitol | 1.5 |
| Aqueous phase | 10 | Propylene glycol | 10.0 |
| | 11 | 2,3-O-(1,3-propanediyl) ascorbic acid (synthesis example 2) | 3.0 |
| | 12 | Preservative | Appropriate amount |
| | 13 | Purified water | Remainder |

### Example 124 Milky lotion

The ingredients (5) to (10) for oil phase and the ingredients (1) to (4) and (11) to (12) for aqueous phase in the compositions shown in Table 24 were heated to 70°C and dissolved, and the oil phase and the aqueous phase were prepared, respectively. The oil phase was then added to the aqueous phase and pre-emulsified, and the mixture was homogeneously emulsified using a homo-mixer. The mixture was then cooled to room temperature while stirring well to prepare a milky lotion.

**[Table 24]**

| | No | Ingredient name | Blend Amount |
|---|---|---|---|
| Aqueous phase | 1 | Dipropylene glycol | 5.0 |
| | 2 | 2,3-O-(1,4-butanediyl) ascorbic acid (synthesis example 3) | 1.0 |
| | 3 | Sorbitan sesquioleate | 4.0 |
| | 4 | Polyoxyethylene monooleate (20) sorbitan | 1.0 |
| Oil phase | 5 | Microcrystalline Wax | 1.0 |
| | 6 | Beeswax | 2.0 |
| | 7 | Lanolin | 2.0 |
| | 8 | Liquid paraffin | 18.0 |
| | 9 | Squalene | 12.0 |
| | 10 | Perfume | Appropriate amount |
| Aqueous phase | 11 | Preservative | Appropriate amount |
| | 12 | Purified water | Remainder |

### Example 125 Cream

The ingredients (1) to (2) for oil phase and the ingredients (3) to (10) for aqueous phase shown in Table 25 are each heated to 70°C and dissolved, and the oil phase and the aqueous phase were prepared, respectively. The oil phase is then added to the aqueous phase for pre-emulsification, and the mixture is homogeneously emulsified using a homo-mixer. The mixture is then cooled to room temperature while stirring thoroughly to prepare a cream.

**[Table 25]**

| | No | Ingredient name | Blend Amount |
|---|---|---|---|
| Oil phase | 1 | Liquid paraffin | 15.0 |
| | 2 | Vaseline | 15.0 |
| Aqueous phase | 3 | Carboxyvinyl polymer | 0.1 |
| | 4 | Xanthan gum | 0.1 |
| | 5 | Polyoxyethylene(40) cured castor oil derivatives. | 3.0 |
| | 6 | 2,3-O-(2-hydroxypropane-1,3-diyl) ascorbic acid (synthesis example 4) | 5.0 |
| | 7 | Sodium hydroxide | 0.05 |
| | 8 | Perfume | Appropriate amount |
| | 9 | Preservative | Appropriate amount |
| | 10 | Purified water | Remainder |

### Example 126 Lotion

A lotion can be prepared by mixing the ingredients (1) to (6) in the composition shown in Table 26 with thorough stirring.

**[Table 26]**

| No | Ingredient name | Blend Amount |
|---|---|---|
| 1 | 2,3-O-(1-hydroxymethyl-1,2-ethanediyl) ascorbic acid (synthesis example 5) | 7.0 |
| 2 | Ethyl alcohol | 8.0 |
| 3 | Citric acid | 0.01 |
| 4 | Sodium citrate | 0.015 |
| 5 | Potassium glycyrrhizinate | 0.03 |
| 6 | Purified water | Remainder |

### Example 127 Cream

The ingredients (1) to (6) and ingredients (7) to (10) shown in Table 27 are each heated to 70°C and dissolved. The oily phase is added to the aqueous phase and pre-emulsified. The mixture is then emulsified using a homo-mixer, and cooled to room temperature while stirring thoroughly to prepare the cream.

**[Table 27]**

| | No | Ingredient name | Blend Amount |
|---|---|---|---|
| Oil phase | 1 | Cetyl alcohol | 2.0 |
| | 2 | Stearyl alcohol | 3.0 |
| | 3 | Squalene | 7.5 |
| | 4 | Tri-2-ethylhexanoate glyceryl | 7.5 |
| | 5 | Methyl polysiloxane | 5.5 |
| | 6 | 2,3-O-(3,3-dimethyleneoxetane) ascorbic acid (synthesis example 6) | 3.0 |
| Aqueous phase | 7 | 1,3-Butylene glycol | 5.0 |
| | 8 | Hydroxyethyl cellulose | 0.2 |
| | 9 | Preservative | Appropriate amount |
| | 10 | Purified water | Remainder |

### Example 128 Cream

The ingredients (1) to (5) for oil phase and the ingredients (6) to (10) for aqueous phase in the compositions shown in Table 28 are each heated to 70°C and dissolved to prepare an oil phase and an aqueous phase, respectively. The oil phase is then added to the aqueous phase and pre-emulsified, and the mixture is homogeneously emulsified using a homo-mixer. The mixture is then cooled to room temperature while stirring thoroughly to prepare a cream.

**[Table 28]**

| | No | Ingredient name | Blend Amount |
|---|---|---|---|
| Oil phase | 1 | Squalene | 9.0 |
| | 2 | Vaseline | 6.0 |
| | 3 | Stearyl alcohol | 5.0 |
| | 4 | Polyoxyethylene(25) cetyl ether | 2.5 |
| | 5 | Glyceryl monostearate | 1.5 |
| Aqueous phase | 6 | 5,6-O-diisostearoyl-2,3-O-(1,4-butanediyl) ascorbic acid (synthesis example 18) | 2.0 |
| | 7 | Glycerin | 6.0 |
| | 8 | Preservative | Appropriate amount |
| | 9 | pH Adjuster | Appropriate amount |
| | 10 | Purified water | Remainder* |

### Example 129 Cream

The ingredients (1) to (2) for oil phase and the ingredients (3) to (10) for aqueous phase shown in Table 29 are each heated to 70°C and dissolved to prepare an oil phase and an aqueous phase, respectively. The oil phase is then added to the aqueous phase and pre-emulsified, and the mixture is homogeneously emulsified using a homo-mixer. The mixture is then cooled to room temperature while stirring thoroughly to prepare a cream.

**[Table 29]**

| | No | Ingredient name | Blend Amount |
|---|---|---|---|
| Oil phase | 1 | Liquid paraffin | 15.0 |
| | 2 | Vaseline | 15.0 |
| Aqueous phase | 3 | Carboxyvinyl polymer | 0.1 |
| | 4 | Xanthan gum | 0.1 |
| | 5 | Polyoxyethylene(40) cured castor oil derivatives. | 3.0 |
| | 6 | 6-O-isostearoyl-2,3-O-(1,3-propanediyl) ascorbic acid (synthetic example 28) | 5.0 |
| | 7 | Sodium hydroxide | 0.05 |
| | 8 | Perfume | Appropriate amount |
| | 9 | Preservative | Appropriate amount |
| | 10 | Purified water | Remainder |

## Claims

1. An ascorbic acid derivative which is represented by the following general formula (1), (3) or (4): 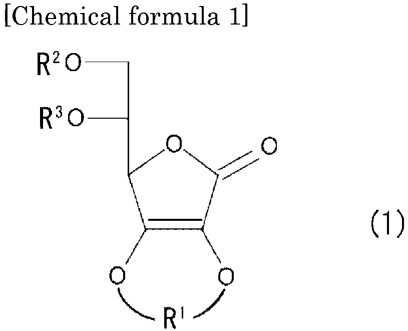 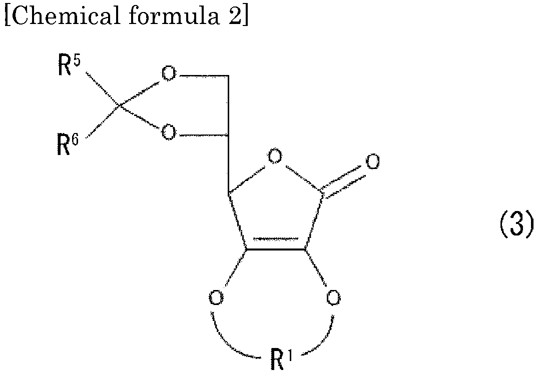 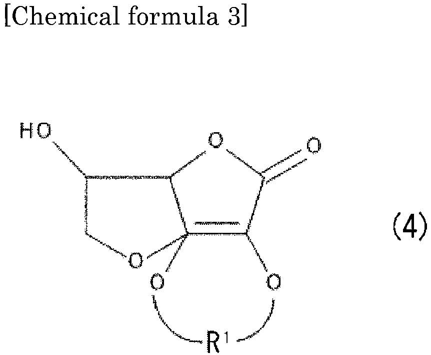 [In formula (1), (3) or (4), R¹ is -(CH₂)ₙ -* (n is an integer of 2 to 4), - CH₂ CH(OH)CH₂ -*, -CH₂ CH(CH₂ OH)-*, -CH(CH₂ OH)CH₂ -*, -C(CH₃ )₂ CH₂-*, -CH₂ C(CH₃ )₂ -* (* represents the position bonding to O at the 2-position of the ascorbic ring), or a divalent group represented by the following formula (2);
in formula (1), R² and R³ are each hydrogen, -COR⁴, a linear or branched alkyl group having 1 to 22 carbon atoms, or a benzyl group, and R⁴ is a linear or branched alkyl group having 1 to 22 carbon atoms; and
in formula (3), R⁵ and R⁶ are each a hydrogen atom, a methyl group, or a phenyl group.]

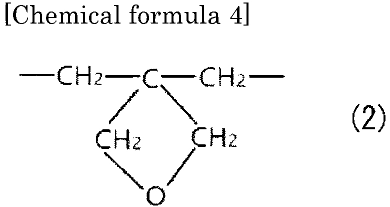

2. An ascorbic acid derivative according to Claim 1, which is represented by the general formula (1), wherein R¹ in formula (1) is -(CH₂) ₙ-* (n is 3 or 4), -CH₂CH( OH)CH₂-*, -CH₂CH(CH₂OH) - *, -CH(C H ₂ O H )CH₂-* (* represents the position bonding to the O at the 2-position of the ascorbic acid ring), or a divalent group represented by formula (2), and R² and R³ are hydrogen; or R¹ is -(CH₂) ₙ-* (n is 3 or 4), R² is -COR⁴, and R³ is hydrogen or-COR⁴, and R⁴ is a linear or branched alkyl group having 4 to 18 carbon atoms.

3. An ascorbic acid derivative according to Claim 2, which is represented by the general formula (1), wherein R¹ in general formula (1) is-(CH₂) ₃-* , -CH₂CH(OH)CH₂-* (* represents the position bonding to the O at position 2 of the ascorbic acid ring), or a divalent group represented by the formula (2), and R² and R³ are each hydrogen, or R¹ is -(CH₂)₃-*, R² is -COR⁴, R³ is hydrogen or -COR⁴, and R⁴ is a linear or branched alkyl group having 8 to 18 carbon atoms.

4. A cosmetics containing the ascorbic acid derivative according to any one of claims 1 to 3.

5. A collagen production promoter comprising the ascorbic acid derivative according to claim 3.

6. A hyaluronic acid production promoter comprising the ascorbic acid derivative according to any one of claims 1 to 3.

7. A hyaluronic acid production promoter comprising the ascorbic acid derivative according to claim 3.
